(19) Europäisches Patentamt
European Patent Office
Office européen des brevets

(11) **EP 4 197 466 B1**

(12) **EUROPÄISCHE PATENTSCHRIFT**

(45) Veröffentlichungstag und Bekanntmachung des Hinweises auf die Patenterteilung:
**15.01.2025   Patentblatt 2025/03**

(21) Anmeldenummer: **22207515.2**

(22) Anmeldetag: **17.12.2014**

(51) Internationale Patentklassifikation (IPC):
*A61B 17/80* (2006.01)        *A61B 17/86* (2006.01)
*A61B 17/68* (2006.01)        *A61F 2/28* (2006.01)

(52) Gemeinsame Patentklassifikation (CPC):
**A61B 17/683; A61B 17/8052; A61B 17/8071; A61B 17/8085; A61B 17/8635; A61B 17/8645; A61F 2/2803;** A61B 17/8057; A61B 2017/8655; A61F 2002/2807

(54) **KNOCHENPLATTE, CHIRURGISCHE SETS UND REKONSTRUKTIONSSETS**

BONE PLATE, SURGICAL SETS AND RECONSTRUCTION SETS

PLAQUE OSSEUSE, ENSEMBLES CHIRURGICAUX ET ENSEMBLES DE RECONSTRUCTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.06.2023   Patentblatt 2023/25**

(62) Dokumentnummer(n) der früheren Anmeldung(en) nach Art. 76 EPÜ:
**19203806.5 / 3 616 636**
**14816223.3 / 3 232 962**

(73) Patentinhaber: **Medartis Holding AG**
**4057 Basel (CH)**

(72) Erfinder:
• **THIEL, Dirk**
  **79219 Staufen (DE)**
• **MULLIS, Andreas**
  **4456 Tenniken (CH)**
• **SCHONHARDT, Jürgen**
  **79618 Rheinfelden (DE)**
• **ZEUNER, Hermann**
  **79111 Freiburg (DE)**
• **SCHÄTZLE, Simon Martin**
  **79288 Gottenheim (DE)**

(74) Vertreter: **Hepp Wenger Ryffel AG**
**Friedtalweg 5**
**9500 Wil (CH)**

(56) Entgegenhaltungen:
EP-A1- 1 468 656        EP-A2- 1 107 699
WO-A1-2004/086990        US-A- 6 129 728
US-A1- 2007 238 069        US-A1- 2009 281 543
US-A1- 2009 312 802        US-A1- 2011 144 698

**Beschreibung**

Die vorliegende Erfindung befasst sich mit Knochenplatten gemäß dem Oberbegriff des Anspruchs 1. Knochenschrauben und chirurgische Sets, die solche Knochenplatten und Knochenschrauben enthalten, werden ebenfalls beschrieben und beansprucht.

Derartige Knochenplatten können zur Überbrückung eines Knochendefekts bzw. zur Versorgung einer Fraktur eingesetzt werden. Insbesondere befasst sich die Erfindung mit Knochenplatten für die Rekonstruktion oder Traumabehandlung eines menschlichen Unterkiefers.

Der Aspekt der Erfindung betrifft eine Knochenplatte, enthaltend mindestens eine Öffnung zur Aufnahme jeweils mindestens einer Knochenschraube, wobei die Öffnung die Knochenplatte entlang einer Längsachse von einer Oberseite zu einer gegenüberliegenden Unterseite durchdringt, wobei die Öffnung an der Oberseite in einen ersten Aufnahmebereich mündet, der zur Aufnahme und Fixierung eines Verblockungselementes einer Knochenschraube in einer ersten Richtung ausgebildet ist.

Eine Knochenplatte mit diesen Merkmalen des Oberbegriffs ist aus der WO 2004/086990 A1 bekannt.

Eine weitere Knochenplatte ist aus der US 2009/281543 A1 bekannt.

Bei der Versorgung von instabilen Trümmerfrakturen, bei Rekonstruktionen mit Knochentransplantaten und insbesondere bei der Überbrückung von Knochendefekten ohne Knochentransplantat werden stabile Knochenplatten benötigt, mit denen die im Defektbereich auftretenden Lasten sicher aufgenommen werden. Eine gattungsgemässe Knochenplatte für einen menschlichen Unterkiefer ist beispielsweise aus der WO 01/82809 bekannt. Mit derartigen Platten können beispielsweise geschwächte, zurückgebildete Knochenstrukturen eines Unterkiefers verstärkt werden. Solche Knochenstrukturen können etwa bei der Entfernung eines Tumors oder auch durch eine Verletzung entstehen, zum Beispiel durch einen Schuss. Die Knochenplatte soll über einen langen Zeitraum hinweg den täglichen Belastungen standhalten können, die beispielsweise beim Kauen oder Schlucken auftreten. Damit sich die Knochenplatte hierbei nicht merklich verbiegt, muss sie eine gewisse Steifigkeit aufweisen. Zu diesem Zweck wird üblicherweise ein hartes Material verwendet, wie beispielsweise Titan vom Grade 4. Titan dieses Grades ist einerseits sehr hart und damit steif gegenüber Biegungen, andererseits jedoch auch relativ spröde.

Die Anatomie menschlicher Knochen und insbesondere eines menschlichen Unterkiefers ist allerdings individuell sehr verschieden. So variiert etwa der Abstand des Kieferwinkels, der zwischen aufsteigendem Asts und Horizontalast gebildet ist, zum Mittelpunkt des Kinns, beispielsweise in Abhängigkeit von Alter, Grösse und Geschlecht des Patienten. Durch die individuell auftretenden Variationen der Knochenform, im Besonderen am menschlichen Unterkiefer, werden anatomisch anformbare Platten oder speziell angefertigte patientenspezifische Platten benötigt. Aus praktischer Sicht ist es daher für ein Krankenhaus oder einen Chirurgen kaum möglich, für jede Anatomie eine vorgeformte, passende Knochenplatte auf Lager zu halten. Stattdessen muss der Chirurg die Knochenplatte durch Schneiden und Biegen an die individuelle Anatomie des Patienten anpassen.

Bisher werden unterschiedliche Therapieansätze verfolgt. Die drei wichtigsten Behandlungsmethoden versorgen die beschriebenen Defekte mit anformbaren Miniplatten, mit speziellen anformbaren Rekonstruktionsplatten und, wie bereits erwähnt, mit speziell angefertigten patientenspezifischen Platten.

Miniplatten, wie beispielsweise die in WO 00/66012 A1 und WO 03/068091 A1 offenbaren, sind Knochenplatten mit einer relativ geringen Materialstärke. Sie haben den Vorteil, dass sie als Standardplatten für einfache und gut reponierbare Frakturen bereits verbreitet Anwendung finden und speziell am Unterkiefer flexibel eingesetzt werden können. Die Miniplatten lassen sich mit einfachen Biegeinstrumenten leicht an die Knochenfragmente anformen. Allerdings weisen Miniplatten aufgrund ihrer Materialstärke besonders bei höherer Belastung gewisse Grenzen auf. Der Einsatz bei den oben beschriebenen Indikationen führt oft postoperativ zu Plattenbrüchen und Dislokationen.

Hier liegen die Vorteile der Rekonstruktionsplatten: Rekonstruktionsplatten sind relativ massive Platten, typischerweise aus einem harten Titan-Grade oder Titanlegierungen mit einer Materialstärke von 2,0 bis 3,5 mm, die sehr stabil sind und hohe Kräfte aufnehmen können. Diese Rekonstruktionsplatten sind üblicherweise so ausgebildet, dass die Biegebereiche zwischen den Befestigungsöffnungen liegen, wobei die Öffnungen und Biegebereiche perlenkettenartig aneinander gereiht sind. Durch die Biegebereiche kann die Platte, von Öffnung zu Öffnung, anatomisch an die Knochenfragmente angepasst werden.

Rekonstruktionsplatten lassen sich jedoch trotz ihrer speziell ausgebildeten Verformungsbereiche nur schwer an die jeweilige Knochenform anpassen, insbesondere im Bereich des aufsteigenden Astes eines Unterkiefers. Zum Anbiegen der Platten werden aufgrund der Materialstärke und der Materialfestigkeit sehr hohe Kräfte benötigt. Die Biegewerkzeuge müssen aufgrund der grossen erforderlichen Kräfte sehr massiv, passgenau und mit entsprechenden Hebelwegen ausgebildet sein.

Das Anformen der Platten ist zudem sehr zeitaufwendig und erfordert sehr viel Erfahrung seitens der Anwender. Denn insbesondere können die Knochenplatten nicht direkt am Unterkiefer des Patienten angeformt werden. Da nämlich die Knochenplatte aus den oben genannten Gründen eine gewisse Steifigkeit aufweisen muss, müssten sehr grosse Kräfte angewendet werden, die den bereits bestehenden Knochendefekt noch weiter verstärken könnten. Der Chirurg muss also zunächst die individuelle Anatomie des Unterkiefers grob ausmessen und die Knochenplatte dann mehr oder weniger nach Augenmass ausserhalb des Patienten zurechtbiegen, was üblicherweise mithilfe einer oder mehrerer Biegezangen geschieht. Alternativ können auch sogenannte Templates, also weiche Metallplatten, verwendet werden. Diese werden wie eine Schablone an den Kiefer angebogen, und die zu biegende Knochenplatte wird anhand dieser Schablone wiederum ausserhalb des Patienten in der beschriebenen Art und Weise gebogen.

Ein solches Biegen mit Hilfe von Biegezangen ist allerdings im Allgemeinen nicht sehr präzise. Dies gilt insbesondere, da die Knochenplatte im Allgemeinen nicht in einer Ebene verlaufen soll. Es kann also passieren, dass die zurechtgebogene Knochenplatte nicht oder nur unzureichend an den Unterkiefer angepasst ist. In diesem Falle muss der Chirurg mehrere Anpassungsschritte vornehmen. Aufgrund der inhärenten Sprödigkeit des Materials (beispielsweise Titan vom Grade 4) ist dies aber nur wenige Male möglich. Es könnte nämlich die Gefahr bestehen, dass das Material der Knochenplatte durch das mehrfache Biegen derart geschwächt wird, dass es entweder bereits während der Operation oder postoperativ im Patienten brechen kann.

Ferner kann es aufgrund der Anformung zu Kaltverfestigungen und Mikrorissen kommen. Speziell bei Platten für den Unterkiefer kann durch die stark variierenden Winkel zwischen Corpus und aufsteigendem Ast eine hohe Verformung der Platten im Kieferwinkelbereich notwendig sein. Dies verstärkt die oben aufgeführte Mikrorissbildung und führt oft postoperativ zu Plattenbrüchen vermehrt im Bereich der starken Verformungen.

Hinzu kommt, dass durch die Aneinanderreihung der Öffnungen die fraktur- bzw. defektnahe Knochenschraube die grösste Last aufnehmen muss. Aufgrund dieser punktuell hohen Last kommt es postoperativ oft zu Schraubenbrüchen oder Osteonekrosen im Bereich der fraktur- bzw. defektnahe Knochenschraube. Speziell bei Überbrückungen von knöchernen Defekten treten durch die sägezahnänlichen Aussenkonturen der Rekonstruktionsplatten oft Weichteilreizungen oder sogar Weichteilnekrosen im Bereich der Überbrückung auf.

Zudem kann es beim Verbiegen einer beispielsweise wie in WO 01/82809 offenbarten Knochenplatte mit bekannten Biegezangen auch zu unerwünschten Verbiegungen innerhalb der Plattenebene oder zu unerwünschten Torsionen kommen.

Patientenspezifisch angefertigte Implantate können die oben erwähnten Nachteile der Miniplatten und Rekonstruktionsplatten beseitigen, sind jedoch sehr aufwendig in der Planung und Vorbereitung, so dass eine gewisse Anzahl von Fachleuten für die Herstellung erforderlich ist. Hinzu kommt, dass patientenspezifisch angefertigte Implantate momentan nur für planbare Eingriffe sinnvoll eingesetzt werden können und relativ teuer im Vergleich zu in Serie gefertigten Platten sind.

Die US 4,726,808 offenbart Unterkieferplatten mit einem mittleren Abschnitt und zwei sich davon erstreckenden Flügeln, die an gegenüberliegenden Seiten eines Ramus angeordnet werden können. In einem mittleren Abschnitt ist die Prothese im Wesentlichen streifenförmig ausgebildet und enthält Öffnungen für Knochenschrauben, die entlang einer Mittellinie des mittleren Abschnittes angeordnet sind. Die Anordnung der Flügel an gegenüberliegenden Seiten eines Ramus ist jedoch aufwendig. Zudem sind diese Platten nur schwer an die individuelle Anatomie eines Patienten anzuformen, insbesondere im Bereich des aufsteigenden Astes. Um also für möglichst jeden Defekt eine geeignete Knochenplatte bereitstellen zu können, ohne die oben erwähnten Risiken in Kauf nehmen zu müssen (wie beispielsweise die Gefahr von Mikrorissen bei zu starker Deformation) muss stets ein grosses Sortiment an verschiedenen Knochenplatten bereitgehalten werden, was aufwendig und kostenintensiv ist.

Das Dokument RU 2 033 105 C1 offenbart eine Knochenplatte zur Behandlung von Unterkieferfrakturen im Bereich der Symphyse. Die in den Figuren gezeigte Knochenplatte weist eine Art von Fachwerkstruktur auf. Eine Fixierung bis in den Bereich des Ramus hinein ist mit dieser Knochenplatte jedoch nicht möglich. Ferner ist auch diese Knochenplatte nur schwer an die individuelle Anatomie eines Patienten anzuformen, so dass stets ein grosses Sortiment an verschiedenen Knochenplatten bereitgehalten werden muss.

Die WO 2010/080511 A1 offenbart dreidimensionale Fachwerkimplantate (jedoch keine gattungsgemässen Knochenplatten), die beispielsweise einen Teil eines Unterkiefers ersetzen können. Eine einfache Anpassbarkeit an die individuelle Anatomie eines Patienten ist aber auch hier nicht gegeben, so dass stets ein grosses Sortiment an verschiedenen Knochenplatten bereitgehalten werden muss. Des Weiteren sind diese Fachwerkimplantate nicht dazu ausgelegt, vor allem im Bereich des aufsteigenden Astes den innerhalb der Plattenebene auftretenden Biegekräften standzuhalten, die beispielsweise beim Kauen oder Schlucken auftreten.

Auch die WO 2013/096592 A1 offenbart Vorrichtungen und Verfahren zum Fördern von Knochenwachstum, die eine trabekelartige Gitterstruktur aufweisen, die das Knochenwachstum fördern soll. Gattungsgemässe Knochenplatten sind also nicht offenbart. Zudem mangelt es auch hier an einer einfachen Anpassbarkeit an die individuelle Anatomie eines Patienten. Weiterhin sind diese Strukturen nicht dazu ausgelegt, vor allem im Bereich des aufsteigenden Astes den innerhalb der Plattenebene auftretenden Biegekräften standzuhalten, die beispielsweise beim Kauen oder Schlucken auftreten.

Das Dokument DE 103 35 281 A1 offenbart eine Gitteranordnung für die Osteosynthese. Die in den Figuren 1 bis 3 gezeigten Knochenplatten enthalten Doppelreihen von Schraubenlöchern, die jeweils in einer geraden Linie angeordnet sind und über dazwischen diagonal verlaufende Stege zu einer flächigen Gitteranordnung verbunden sind. Auch diese Gitteranordnung ist jedoch nur schwer an die individuelle Anatomie eines Patienten anzuformen. Ferner sind diese Gitter nicht dazu ausgelegt, vor allem im Bereich des aufsteigenden Astes den innerhalb der Plattenebene auftretenden Biegekräften standzuhalten, die beispielsweise beim Kauen oder Schlucken auftreten. Zudem weisen etwa die Aussenränder der ringförmigen Aufnahmen für die Knochenschrauben Kanten auf, die für den Patienten unangenehm sind und sogar zu einer Verletzung des Körpergewebes führen könnten.

Die EP 1 182 972 B1 offenbart osteosynthetische Knochenplatten zur Behandlung von Frakturen, insbesondere von UnterkieferFrakturen. Auch diese Knochenplatten sind nur schwer an die individuelle Anatomie eines Patienten anzuformen, ohne dass hierfür ein grosses Sortiment an verschiedenen Knochenplatten bereitgehalten werden muss.

Angesichts der vorstehend beschriebenen Nachteile ist die Aufgabe der vorliegenden Erfindung, eine Knochenplatte bereitzustellen, welche diese Nachteile nicht aufweist. Insbesondere soll die Knochenplatte leicht an die individuelle Anatomie eines Patienten anformbar sein, ohne dass hierfür ein übermässig grosses Sortiment an verschiedenen Knochenplatten bereitgehalten werden muss oder die Gefahr einer Mikrorissbildung oder Kaltverfestigung durch zu grosse Deformationen entsteht. Bevorzugt soll die Knochenplatte ferner in den lasttragenden Bereichen frakturnah die erforderliche Stabilität aufbringen und die Lasten im Verankerungsbereich auf mehrere Knochenschrauben verteilen können. Des Weiteren soll die Knochenplatte bevorzugt möglichst dünn sein und im Fraktur- bzw. Überbrückungsbereich mit einer weichteilschonenden Aussenkontur versehen sein.

Aufgabe der vorliegenden Erfindung, die aus dem Stand der Technik bekannten Knochenplatte dahingehend weiterzuentwickeln, dass sie es ermöglicht, wahlweise die Oberseite oder die Unterseite der Knochenplatte an einen Knochen anzulegen und in beiden Orientierungen ein und dieselbe Knochenschraube aufzunehmen und deren Verblockungselement zu fixieren. Diese Aufgabe wird durch eine Knochenplatte mit den Merkmalen des Anspruchs 1 gelöst. Vorteilhafte Weiterbildungen der Erfindung sind Gegenstand der abhängigen Ansprüche. Insbesondere ist ein chirurgisches Set, enthaltend mindestens eine Knochenplatte sowie mindestens eine Knochenschraube Gegenstand des Anspruchs 4.

Der in der vorliegenden Beschreibung verwendete Begriff "Ausführungsform" bzw. "Ausführungsbeispiel" gibt nicht unbedingt Möglichkeiten zur Ausführung der beanspruchten Erfindung an, sondern enthält auch Beispiele, die zum Verständnis der Erfindung beitragen.

Diese Aufgabe werden zum einen gelöst durch eine Knochenplatte für die Rekonstruktion oder Traumabehandlung eines Knochens. Insbesondere kann es sich um einen menschlichen Knochen wie etwa einen menschlichen Unterkiefer handeln. Alternativ können die Knochen aber auch beispielsweise Talus-Naviculare-Cuneiforme-Metatarsale I bei einer "medial column OP" im Fuss sein.

Die Knochenplatte enthält einen Hauptabschnitt mit einem ersten Ende und einem zweiten Ende, einer ersten Anlegefläche zum Anlegen und Befestigen an einem ersten Bereich des Knochens und einer Mehrzahl von Aufnahmen mit jeweils mindestens einer insbesondere kreisförmigen Öffnung zur Aufnahme jeweils mindestens eines Befestigungselementes. Der erste Bereich des Knochens kann etwa der Corpus des Unterkiefers sein. Sind die zu versorgenden Knochen alternativ die Knochen für eine "medial column OP" im Fuss, so kann der erste Bereich aus einem oder mehreren der Knochen Talus, Naviculare, Cuneiforme und Metatarsale I bestehen. Das genannte Befestigungselement ist bevorzugt eine Knochenschraube.

Mindestens vom ersten Ende des Hauptabschnitts erstrecken sich mindestens zwei nebeneinander angeordnete Flügel. Die Formulierung "nebeneinander angeordnet" bedeutet hierbei, dass die Flügel innerhalb ein und derselben im Wesentlichen ebenen Fläche oder innerhalb von zwei im Wesentlichen ebenen Flächen verlaufen, deren Abstand kleiner ist als die Dicke der Flügel, oder dass die Flügel zumindest derart deformierbar sind, dass sie innerhalb ein und derselben im Wesentlichen ebenen Fläche oder innerhalb von zwei im Wesentlichen ebenen Flächen verlaufen, deren Abstand kleiner ist als die Dicke der Flügel - insbesondere im Gegensatz zu den in US 4,726,808 dargestellten Flügeln, die in Ebenen verlaufen, welche einen deutlich grösseren Abstand voneinander aufweisen.

Die sich vom ersten Ende des Hauptabschnittes erstreckenden Flügel verlaufen bevorzugt unter einem Winkel zueinander, der kleiner als 90°, bevorzugt kleiner als 60° und besonders bevorzugt kleiner als 45° ist. Diese Winkelbereiche haben sich insbesondere für die Behandlung eines menschlichen Unterkiefers als vorteilhaft erwiesen.

Die Flügel weisen jeweils eine zweite Anlegefläche zum Anlegen und Befestigen an einen zweiten Bereich des Knochens auf, sowie mindestens eine Aufnahme mit jeweils mindestens einer insbesondere kreisförmigen Öffnung zur Aufnahme jeweils mindestens eines Befestigungselementes. Der zweite Bereich des Knochens kann beispielsweise der aufsteigende Ast eines Unterkiefers sein, insbesondere eine Aussenseite dieses aufsteigenden Astes. Sind die Knochen alternativ die Knochen für eine "medial column OP" im Fuss, so kann der zweite Bereich aus einem oder mehreren der Knochen Talus, Naviculare, Cuneiforme und Metatarsale I bestehen. Auch die Aufnahmen der Flügel können zur Aufnahme jeweils mindestens einer Knochenschraube ausgebildet sein.

Es liegt ebenfalls im Rahmen der Offenbarung, dass sich nicht nur vom ersten Ende des Hauptabschnitts mindestens zwei nebeneinander angeordnete Flügel erstrecken, sondern dass sich auch vom zweiten Ende des Hauptabschnitts mindestens ein Flügel oder auch mindestens zwei nebeneinander angeordnete Flügel erstrecken, der/die die oben genannten Eigenschaften aufweist/aufweisen. Der Hauptabschnitt und die mindestens zwei Flügel sind derart ausgebildet, dass der Hauptabschnitt bezüglich einer senkrecht zur ersten Anlegefläche verlaufenden Achse über eine erste minimale Biegesteifigkeit verfügt und jeder der mindestens zwei Flügel bezüglich einer senkrecht zur zweiten Anlegefläche verlaufenden Achse über eine jeweilige zweite minimale Biegesteifigkeit verfügt. Ist der Hauptabschnitt oder mindestens einer der Flügel gekrümmt oder verläuft der Hauptabschnitt unter einem Winkel zu mindestens einem der Flügel, so sind die genannten senkrecht verlaufenden Achsen an verschiedenen Punkten der Anlegefläche des Hauptabschnitts bzw. des Flügels nicht notwendigerweise parallel zueinander. Hier und im Folgenden wird also stets von lokalen senkrecht zur Anlegefläche verlaufenden Achsen ausgegangen.

Gemäss dem fachüblichen Verständnis gibt die Biegesteifigkeit an, wie gross das Biegemoment im Verhältnis zu der dadurch hervorgerufenen Krümmung ist. Für ein homogenes Material ergibt sich die Biegesteifigkeit als Produkt aus dem Elastizitätsmodul des Materials der Knochenplatte (im Bereich des Hauptabschnitts bzw. eines der Flügel) und dem axialen Flächenträgheitsmoment bezüglich einer durch die Knochenplatte verlaufenden Querschnittsebene (also durch den Hauptabschnitt bzw. durch einen der Flügel).

Für die vorliegende Erfindung werden nur die Querschnittsebenen berücksichtigt, die senkrecht zur jeweiligen Anlegefläche verlaufen, d.h. die parallel zu einer senkrecht auf der Anlegefläche verlaufenden Achse verlaufen. Zur Berechnung des axialen Flächenträgheitsmoments bezüglich einer solche Querschnittsebene wird von einem kartesischen Koordinatensystem ausgegangen, dessen Ursprung durch den Flächenschwerpunkt dieser Querschnittsfläche gebildet wird und dessen x-Achse senkrecht zur Anlegefläche verläuft und zusammen mit der z-Achse die Querschnittsfläche aufspannt. Die y-Achse verläuft somit senkrecht zur Querschnittsfläche und parallel zur Anlegefläche. Rechnerisch lässt sich das axiale Flächenträgheitsmoment durch das Integral

$$\int_A z^2 \, dA$$

der quadrierten z-Koordinate über die Querschnittsfläche A bestimmen. Auf diese Weise ergibt sich die erfindungsgemässe Biegesteifigkeit bezüglich der senkrecht auf der Anlegefläche stehenden x-Achse: Beim Angreifen eines Biegemomentes um die Biegeachse x herum, die senkrecht auf der Anlegefläche steht, erfolgt eine Krümmung um diese Biegeachse x und damit innerhalb der Anlegefläche.

Die genannte Biegesteifigkeit hängt offensichtlich von der Wahl der Querschnittsebene ab, d. h. bei Bezugnahme auf das oben eingeführte Koordinatensystem insbesondere von der Wahl der y-Achse und damit auch der z-Achse, die zusammen mit der senkrecht zur Anlegefläche verlaufenden x-Achse diese Querschnittsebene aufspannt. Als erste minimale Biegesteifigkeit (also als minimale Biegesteifigkeit des Hauptabschnitts) bezüglich einer senkrecht zur ersten Anlegefläche verlaufenden Achse wird hier und im Folgenden die kleinste Biegesteifigkeit bezüglich einer (lokalen) senkrecht zur ersten Anlegefläche verlaufenden Achse angesehen (bei Bezugnahme auf das oben eingeführte Koordinatensystem bezüglich der lokalen x-Achse), die sich aus allen durch den Hauptabschnitt verlaufenden und senkrecht auf der Anlegefläche stehenden Querschnittsebenen ergibt. Analog wird auch die minimale Biegesteifigkeit der Flügel verstanden.

Die erste minimale Biegesteifigkeit (also die minimale Biegesteifigkeit des Hauptabschnitts) ist grösser als jede der zweiten minimalen Biegesteifigkeiten (also der minimalen Biegesteifigkeiten der Flügel), jedoch kleiner als die minimale Gesamtbiegesteifigkeit aller sich vom ersten Ende erstreckenden Flügel. Falls sich nicht nur vom ersten Ende des Hauptabschnitts mindestens zwei nebeneinander angeordnete Flügel erstrecken, sondern sich auch vom zweiten Ende des Hauptabschnitts mindestens zwei nebeneinander angeordnete Flügel erstrecken, so kann die erste minimale Biegesteifigkeit (also die minimale Biegesteifigkeit des Hauptabschnitts) auch grösser als jede der zweiten minimalen Biegesteifigkeiten der sich vom zweiten Ende des Hauptabschnitts erstreckenden Flügel, jedoch kleiner als die minimale Gesamtbiegesteifigkeit aller sich vom zweiten Ende erstreckenden Flügel sein.

Als zweite minimale Gesamtbiegesteifigkeit bezüglich einer senkrecht zur zweiten Anlegefläche verlaufenden Achse wird analog zur obigen Definition die minimale Biegesteifigkeit bezüglich einer (lokalen) senkrecht zur zweiten Anlegefläche verlaufenden Achse verstanden, die sich aus allen senkrecht auf der zweiten Anlegefläche stehenden Querschnittsebenen ergibt, wobei hierfür jedoch nur diejenigen Querschnittsebenen herangezogen werden, die durch sämtliche sich vom ersten Ende erstreckenden Flügel verlaufen. Für die Berechnung der Gesamtbiegesteifigkeit und des Flächenträgheitsmoments wird von einem gemeinsamen Koordinatenmittelpunkt ausgegangen. Die minimale Gesamtbiegesteifigkeit der Flügel ist also nicht mit der Summe der minimalen Biegesteifigkeiten der einzelnen Flügel gleichzusetzen; die minimale Gesamtbiegesteifigkeit ist nämlich grösser als die Summe der minimalen Biegesteifigkeiten der einzelnen Flügel.

Wie oben bereits angedeutet wurde, ist die durch ein Biegemoment hervorgerufene Krümmung der Knochenplatte proportional zum angreifenden Biegemoment und umgekehrt proportional zur Biegesteifigkeit. Die grösste Krümmung und damit auch die grösste Deformation entstehen daher in dem Bereich der Knochenplatte, in dem die Biegesteifigkeit minimal ist.

Die oben erläuterten Relationen führen zu den folgenden vorteilhaften Biegeeigenschaften: Im noch nicht am Knochen fixierten Ausgangszustand sind die Flügel durch Aufbringen eines vergleichsweise geringen Biegemomentes einzeln innerhalb der Plattenebene biegbar. Da die erste minimale Biegesteifigkeit grösser ist als jede der zweiten minimalen Biegesteifigkeiten, erfolgt in diesem Ausgangszustand die grösste Deformation im Bereich der Flügel. Somit können die Flügel relativ einfach innerhalb der Plattenebene deformiert werden, ohne dass der Hauptabschnitt wesentlich deformiert wird.

Ist die Knochenplatte im implantierten Zustand mit dem Hauptabschnitt und den Flügeln am Knochen fixiert, so sind die Flügel auch relativ zueinander fixiert, so dass in diesem implantierten Zustand die minimale Gesamtbiegesteifigkeit relevant ist. Da diese grösser ist als die erste minimale Biegesteifigkeit, können im implantierten Zustand die Flügel kaum noch innerhalb der Plattenebene deformiert werden. Ist der zu behandelnde Knochen ein Unterkiefer, so ist die Forderung, dass die Gesamtbiegesteifigkeit grösser ist als die erste Biegesteifigkeit, der realen Kraftverteilung im Unterkiefer geschuldet: Gelenksnah ist die Belastung auf den Knochen beim Kauen/Beissen grösser als zum Kinn hin.

Die Biegesteifigkeit des Hauptabschnitts bezüglich einer parallel zur Anlegefläche verlaufenden Achse, die die Krümmung des Hauptabschnitts aus der Plattenebene heraus bestimmt (gemäss obiger Definition also bei einer Biegung um die z-Achse), ist hingegen bevorzugt kleiner als die minimale Biegesteifigkeit des Hauptabschnitts bezüglich der Achse, die die Krümmung innerhalb der Plattenebene bestimmt (gemäss obiger Definition also bei einer Biegung um die x-Achse). Gleiches gilt analog auch für die Flügel. Dies ermöglicht es, den Hauptabschnitt bzw. die Flügel aus der Plattenebene heraus zu biegen.

Wie sich gezeigt hat, genügt es für die Anpassung an die meisten menschlichen Unterkiefer, wenn bei entsprechendem Design der Platte der Hauptabschnitt der Knochenplatte lediglich aus der durch die Anlegefläche definierten Ebene heraus (bei Bezugnahme auf das obige Koordinatensystem also durch eine Biegung um die z-Achse), aber nicht innerhalb dieser Ebene (bei Bezugnahme auf das obige Koordinatensystem also um die x-Achse) gebogen wird. Ein Biegen innerhalb der durch die Anlegefläche definierten Ebene würde zudem die Gefahr von Kaltverfestigungen erhöhen.

Die grössten Unterschiede zwischen menschlichen Unterkiefern bestehen stattdessen im Winkel, unter dem sich der aufsteigende Ast gegenüber dem Corpus erstreckt, in der Höhe des aufsteigenden Astes, in der Länge des Corpus. Die hierfür vorgesehenen Flügel sind nicht nur aus der durch die Anlegefläche definierten Ebene heraus, sondern auch innerhalb der Plattenebene leicht verbiegbar - jedenfalls solange sie noch nicht am Unterkiefer befestigt sind.

Für die Feststellung, ob eine gegebene Knochenplatte die oben erläuterten Biegeeigenschaften erfüllt, ist es nicht zwingend erforderlich, die erste minimale Biegesteifigkeit des Hauptabschnittes, die zweiten minimale Biegesteifigkeiten der Flügel und die minimale Gesamtbiegesteifigkeit der Flügel nummerisch exakt zu kennen. Vielmehr genügt es für die Erfüllung der Biegeeigenschaften,

- dass eine erste untere Schranke für die Biegesteifigkeiten bekannt ist, die kleiner ist als alle Biegesteifigkeiten bezüglich lokalen senkrecht zur ersten Anlegefläche verlaufenden Achsen (unter Bezugnahme auf das oben eingeführte Koordinatensystem bezüglich der lokalen x-Achsen), die sich aus den durch den Hauptabschnitt verlaufenden und senkrecht auf der ersten Anlegefläche stehenden Querschnittsebenen ergeben;

- dass jeder der Flügel bezüglich wenigstens einer durch diesen Flügel verlaufenden und senkrecht auf der zweiten Anlegefläche stehenden Querschnittsebene über eine Biegesteifigkeit bezüglich einer lokalen senkrecht zur zweiten Anlegefläche verlaufenden Achse verfügt, die kleiner ist als die genannte erste untere Schranke;

- dass eine zweite untere Schranke für die Gesamtbiegesteifigkeiten bekannt ist, die kleiner ist als alle Gesamtbiegesteifigkeiten bezüglich lokalen senkrecht zu den zweiten Anlegefläche verlaufenden Achsen, die sich aus den senkrecht auf den zweiten Anlegeflächen stehenden Querschnittsebenen ergeben, wobei hierfür nur diejenigen Querschnittsebenen herangezogen werden, die durch sämtliche sich vom ersten Ende erstreckenden Flügel verlaufen;

- dass der Hauptabschnitt bezüglich wenigstens einer durch den Hauptabschnitt verlaufenden und senkrecht auf der ersten Anlegefläche stehenden Querschnittsebene über eine Biegesteifigkeit bezüglich einer lokalen senkrecht zu den ersten Anlegefläche verlaufenden Achse verfügt (unter Bezugnahme auf das oben eingeführte Koordinatensystem bezüglich der lokalen x-Achse), die kleiner ist als die genannte zweite untere Schranke.

Um eine Deformation des Hauptabschnittes innerhalb der Anlegefläche möglichst zu reduzieren, kann der Hauptabschnitt eine Fachwerkstruktur aufweisen. Dies bedeutet, dass der Hauptabschnitt über Verstrebungen verfügt, die transversal zu einer sich vom ersten Ende zum zweiten Ende des Hauptabschnitts erstreckenden Mittellinie verlaufen, also senkrecht und/oder parallel und/oder bevorzugt diagonal zu dieser Mittellinie. Vorteilhafterweise bilden die Aufnahmen Knotenpunkte der Fachwerkstruktur, und die Verstrebungen verlaufen zwischen diesen Aufnahmen. Es sind jedoch auch Fachwerkstrukturen denkbar, bei denen nur einzelne oder keine Aufnahmen Knotenpunkte bilden.

Es ist denkbar, dass auch mindestens ein, bevorzugt sämtliche Flügel ebenfalls eine wie oben beschriebene Fachwerkstruktur enthalten. Bevorzugt weist jedoch mindestens ein Flügel und besonders bevorzugt jeder Flügel keine solche Fachwerkstruktur auf. Auf diese Weise kann erreicht werden, dass zumindest im nicht implantierten Ausgangszustand die Flügel bezüglich einer senkrecht zur jeweiligen Anlegefläche verlaufenden Achse leichter deformierbar sind als der Hauptabschnitt.

Der Hauptabschnitt kann zumindest an einer Seite, bevorzugt an beiden Seiten von mindestens einer Rahmenstruktur begrenzt sein, welche einen äusseren Rand aufweist, der im Wesentlichen geradlinig verläuft. Der äussere Rand wird dabei als im Wesentlichen geradlinig bezeichnet, wenn sein minimaler Krümmungsradius zumindest an den Längsseiten des Hauptabschnitts grösser als 10 mm ist und/oder seine senkrecht zur Mittellinie gemessene Breite auf einer Länge von 10 mm entlang der Mittellinie um nicht mehr als 2 mm variiert. Hierdurch können Kanten vermieden werden, wie sie beispielsweise durch die ringförmigen Schraubenaufnahmen der DE 103 35 281 A1 entstehen und die für den Patienten unangenehm sein können und sogar zu einer Verletzung des Körpergewebes führen könnten. Im Bereich des ersten Endes und des zweiten Endes des Hauptabschnitts und/auch an den Flügeln kann der Krümmungsradius jedoch auch kleiner sein bzw. kann die senkrecht zur Mittellinie gemessene Breite auch mehr variieren.

Der Hauptabschnitt kann eine Breite aufweisen, die im Bereich von 2 mm bis 20 mm, bevorzugt von 5 mm bis 15 mm, besonders bevorzugt von 8 mm bis 10 mm liegt. Die entlang einer Mittellinie gemessene Länge des Hauptabschnitts kann im Bereich von 25 mm bis 300 mm, bevorzugt von 50 mm bis 250 mm liegen. Mindestens ein Flügel, bevorzugt jeder Flügel kann eine Länge aufweisen, welche im Bereich von 10 mm bis 60 mm, bevorzugt von 20 mm bis 40 mm liegt. Ferner kann mindestens ein Flügel, bevorzugt jeder Flügel eine Breite im Bereich von 2 mm bis 10 mm aufweisen. Weiterhin kann mindestens ein Flügel, bevorzugt jeder Flügel eine Breite aufweisen, welche höchstens 80 % der Breite des Hauptabschnitts beträgt. Derartige Dimensionen und Verhältnisse, die auch unabhängig voneinander gewählt werden können, sind jedenfalls für menschliche Unterkiefer geeignet.

Senkrecht zu den Anlegeflächen (also zur ersten Anlegefläche und den zweiten Anlegeflächen) kann die Knochenplatte eine Dicke aufweisen, die im Bereich von 1 mm bis 3 mm, bevorzugt von 1 mm bis 2 mm liegt. Aufgrund der Biegeeigenschaften und/oder der Fachwerkstruktur führen derart geringe Dicken dennoch nicht zu einem Verlust an Biegesteifigkeit.

Bevorzugt ist die Knochenplatte im Wesentlichen eben ausgebildet. Dies bedeutet, dass sowohl der Hauptabschnitt als auch die Flügel im Wesentlichen in einer gemeinsamen Ebene verlaufen (abgesehen von der Ausdehnung von der Knochenplatte aufgrund ihrer oben genannten Dicke). Die obige Knochenplatte aus US 4,726,808 ist nach dieser Definition nicht eben, da ihre beiden Flügel in verschiedenen Ebenen verlaufen. Eine im Wesentlichen ebene Knochenplatte kann einfacher hergestellt werden als eine bereits an die anatomische Form angepasste Knochenplatte. Zudem vereinfacht eine im Wesentlichen ebene Form den Transport und die Lagerung der Knochenplatte.

Ferner ist der Hauptabschnitt bevorzugt im Wesentlichen nur durch ein Biegen aus der durch die Anlegefläche definierten Ebene heraus in eine anatomische Form deformierbar, in welcher er an zumindest einem Teil eines insbesondere menschlichen Unterkiefers befestigbar ist. Mit anderen Worten ist der Hauptabschnitt derart ausgebildet, dass er nicht oder höchstens nur unwesentlich um die senkrecht zur Anlegefläche verlaufenden Achse gebogen werden muss, um in die anatomische Form überführt zu werden. Eine Verbiegbarkeit soll nämlich, wie oben erläutert wurde, reduziert werden, damit die Knochenplatte auch im implantierten Zustand höchstens geringfügig durch Kräfte oder Biegemomente deformierbar ist, die innerhalb der genannten Ebenen wirken.

Die Knochenplatte besteht bevorzugt aus einem biokompatiblen Implantatwerkstoff, wie beispielsweise Titan und seinen Legierungen, Implantatstahl, implantierbarem Kunststoff oder implantierbarer Keramik.

In vielen Situationen ist es vorteilhaft, wenn eine Knochenschraube durch entsprechende Ausgestaltung sowohl eines Verblockungselementes der Knochenschraube, insbesondere des Schraubenkopfes, als auch der Plattenöffnung unter verschiedenen Winkeln relativ zur Knochenplatte fixiert werden kann. Derartige Strukturen an Schraubenköpfen und Plattenöffnungen sind z.B. in der internationalen Patentanmeldung WO 2004/086990 offenbart. Die dort offenbarten Plattenöffnungen erlauben jedoch nur eine winkelvariable Fixierung des Verblockungselementes, insbesondere des Schraubenkopfes, wenn die Schraube von einer vorgegebenen Oberseite der Knochenplatte in Richtung zu einer vorgegebenen gegenüberliegenden Unterseite der Knochenplatte durch die Öffnung eingesetzt wird; eine winkelvariable Fixierung in einer dazu entgegengesetzten Richtung ist nicht möglich. Dies kann die Verwendbarkeit der Knochenplatten für manche Anwendungen einschränken.

Es ist die Aufgabe der vorliegenden Erfindung, die aus dem Stand der Technik bekannten Knochenplatten und insbesondere deren Öffnungen derart weiterzubilden, dass hierfür geeignete Knochenschrauben (insbesondere die in WO 2004/086990 offenbarten Knochenschrauben) in beiden Richtungen winkelvariabel an der Knochenplatte fixiert werden können.

Diese Aufgabe wird gelöst durch eine Knochenplatte mit mindestens einer Öffnung zur Aufnahme jeweils mindestens einer Knochenschraube. Insbesondere kann es sich um eine wie oben beschriebene Knochenplatte für die Rekonstruktion oder Traumabehandlung eines insbesondere menschlichen Knochens, wie etwa eines insbesondere menschlichen Unterkiefers, handeln.

Die Öffnung durchdringt die Knochenplatte entlang einer Längsachse von einer Oberseite der Knochenplatte zu einer gegenüberliegenden Unterseite der Knochenplatte. Die Öffnung mündet an der Oberseite in einen ersten Aufnahmebereich, der zur Aufnahme und insbesondere winkelvariablen Fixierung eines Verblockungselementes einer Knochenschraube in einer ersten Richtung ausgebildet ist.

Erfindungsgemäss mündet die Öffnung an der Unterseite in einen zweiten Aufnahmebereich, der zur Aufnahme und insbesondere winkelvariablen Fixierung des Verblockungselementes in einer zweiten Richtung ausgebildet ist. Dabei ist die zweite Richtung im Wesentlichen entgegengesetzt zur ersten Richtung. Darunter wird hier und im Folgenden verstanden, dass das Verblockungselement derart im ersten Aufnahmebereich aufgenommen und fixiert werden kann, dass die Knochenschraube die Knochenplatte von der Oberseite in Richtung der Unterseite durchdringt, und das Verblockungselement derart im zweiten Aufnahmebereich aufgenommen und fixiert werden kann, dass die Knochenschraube die Knochenplatte von der Unterseite in Richtung der Oberseite durchdringt.

Bevorzugt sind der erste Aufnahmebereich von einer ersten Innenwand begrenzt und der zweite Aufnahmebereich von einer zweiten Innenwand begrenzt, wobei sowohl in der ersten Innenwand als auch in der zweiten Innenwand jeweils mindestens eine Ausnehmung gebildet ist und sich in jeder dieser Ausnehmungen der Abstand der jeweiligen Innenwand in Abhängigkeit vom Umlaufwinkel um die Längsachse weg vergrössert. Noch weiter bevorzugt sind sowohl die erste Innenwand als auch die zweite Innenwand im Bereich jeder der jeweiligen Ausnehmungen zumindest annähernd sphärisch, paraboloidisch, ellipsoidisch oder hyperboloidisch ausgebildet.

Mit anderen Worten enthält die Öffnung also bevorzugt sowohl im Bereich der Oberseite der Knochenplatte als auch im Bereich der Unterseite der Knochenplatte eine Innenwand, wobei diese Innenwände jeweils wie in WO 2004/086990 offenbart beschaffen sind.

Der erste und der zweite Aufnahmebereich der Öffnung können unabhängig voneinander für eine rechtsdrehende Verblockung oder für eine linksdrehende Verblockung ausgebildet sein. Dabei bedeutet eine rechtsdrehende Verblockung beispielsweise für den ersten Aufnahmebereich, dass das Verblockungselement der Knochenschraube, in Blickrichtung von der Oberseite der Knochenplatte zur Unterseite der Knochenplatte betrachtet, durch Drehen der Knochenschraube im Uhrzeigersinn im ersten Aufnahmebereich fixierbar ist. Analog bedeutet eine linksdrehende Verblockung beispielsweise für den zweiten Aufnahmebereich, dass das Verblockungselement, in Blickrichtung von der Oberseite der Knochenplatte zur Unterseite der Knochenplatte betrachtet, durch Drehen der Knochenplatte gegen den Uhrzeigersinn im ersten Aufnahmebereich fixierbar ist.

Bevorzugt sind die erste und die zweite Aufnahmebereiche der Öffnung für eine gleichdrehende Verblockung ausgebildet. Dies bedeutet, dass entweder beide Aufnahmebereiche für eine rechtsdrehende Verblockung oder beide Aufnahmebereiche für eine linksdrehende Verblockung ausgebildet sind.

Diese Ausgestaltung erlaubt es, eine entsprechend ausgestaltete Knochenschraube sowohl von der Oberseite in Richtung der Unterseite durch die Öffnung einzusetzen und so winkelvariabel zu fixieren als auch von der Unterseite in Richtung der Oberseite durch die Öffnung einzusetzen und so winkelvariabel zu fixieren. Dies ermöglicht es, Knochenplatten, insbesondere die oben beschriebenen Knochenplatten, derart auszugestalten, dass wahlweise die Unterseite oder die Oberseite an den Knochen angelegt werden kann. Insbesondere kann ein und dieselbe Knochenplatte wahlweise für einen linksseitigen oder für einen rechtsseitigen Defekt eingesetzt werden. Auf diese Weise sind noch weniger verschiedene Knochenplatten erforderlich, um eine individuelle Anpassung zu erlauben, was die Lagerhaltung nochmals vereinfacht. Die Knochenplatte erlaubt sogar einige Anwendungen, bei denen mindestens eine erste Knochenschraube durch eine erste Öffnung von der Oberseite in Richtung der Unterseite und mindestens eine zweite Knochenschraube durch eine zweite Öffnung von der Unterseite in Richtung der Oberseite ein und derselben Knochenplatte eingesetzt und winkelvaribel fixiert werden können.

Die erste und/oder die zweite Innenwand können, unabhängig voneinander, eines, mehrere oder sämtliche der in WO 2004/086990 offenbarten Merkmale aufweisen.

Insbesondere kann die Innenwand mindestens drei oder sogar genau drei gleichmässig entlang ihres Umfangs verteilte Ausnehmungen aufweisen, die sich jeweils keilförmig nach aussen von der Längsachse der Aufnahme weg erweitern; und/oder der Aufnahmebereich kann mit einer insbesondere sphärischen Ansenkung versehen sein zur Aufnahme beispielsweise eines Schraubenkopfs mit sphärischer Kopfunterseite.

Weiterhin betrifft die Erfindung chirurgisches Set, welches mindestens eine wie oben beschriebene Knochenplatte mit mindestens einer Öffnung mit zwei Aufnahmebereichen enthält sowie mindestens eine Knochenschraube mit einem Schraubenschaft und einem Verblockungselement, insbesondere einem über den Schraubenschaft und ein Gewinde des Schraubenschaftes nach aussen vorstehenden Schraubenkopf. Dabei ist das Verblockungselement wahlweise im ersten Aufnahmebereich oder im zweiten Aufnahmebereich der Öffnung der Knochenplatte aufnehmbar und insbesondere winkelvariabel fixierbar.

Bevorzugt ist das Verblockungselement, insbesondere der Schraubenkopf, mit einer umlaufenden Aussenfläche versehen, die sich im Wesentlichen in Richtung einer Längsachse der Knochenschraube erstreckt und mindestens eine Klemmfläche aufweist, welche sich - in einer Azimutebene senkrecht zur Längsachse betrachtet - keilförmig nach aussen von der Längsachse weg erweitert. Bevorzugt ist die umlaufende Aussenfläche des Verblockungselementes wenigstens im Bereich der Klemmfläche zumindest annähernd sphärisch, paraboloidisch, ellipsoidisch oder hyperboloidisch ausgestaltet.

Mit anderen Worten ist die Knochenschraube bevorzugt so ausgebildet, wie es in WO 2004/086990 offenbart ist.

Eine derart ausgestaltete Knochenschraube erlaubt das wahlweise Einsetzen in die Öffnung der Knochenplatte in im Wesentlichen zueinander entgegengesetzten Richtungen, wie oben bereits erläutert wurde. Auch die Knochenschraube kann eines, mehrere oder sämtliche der in WO 2004/086990 offenbarten Merkmale aufweisen.

Insbesondere kann die Aussenfläche mindestens drei oder sogar genau drei gleichmässig entlang ihres Umfangs verteilte Klemmflächen aufweisen, die sich jeweils keilförmig nach aussen von der Längsachse weg erweitern.

Weiterhin wird ein Rekonstruktionsset für die Rekonstruktion eines insbesondere menschlichen Unterkiefers beschrieben, wobei das Rekonstruktionsset mindestens eine wie oben beschriebene Knochenplatte sowie mindestens eines der folgenden weiteren Elemente aufweist:

- mindestens eine Verbindungsplatte, welche Mittel zum Verbinden mit mindestens einer der Knochenplatten oder einem Teil einer der Knochenplatten aufweist, insbesondere mindestens eine Öffnung zur Aufnahme eines Befestigungselements, insbesondere einer Verbindungsschraube oder einer Knochenschraube,

- mindestens eine Kiefergelenkprothese sowie optional mindestens ein Trägerelement zum Halten der Kiefergelenkprothese, wobei die Kiefergelenkprothese und/oder das Trägerelement Mittel zum Verbinden mit mindestens einem Flügel, bevorzugt allen sich vom ersten Ende des Hauptabschnittes der Knochenplatte erstreckenden Flügeln aufweist, insbesondere mindestens eine Öffnung zur Aufnahme eines Befestigungselementes, insbesondere einer Verbindungsschraube oder einer Knochenschraube.

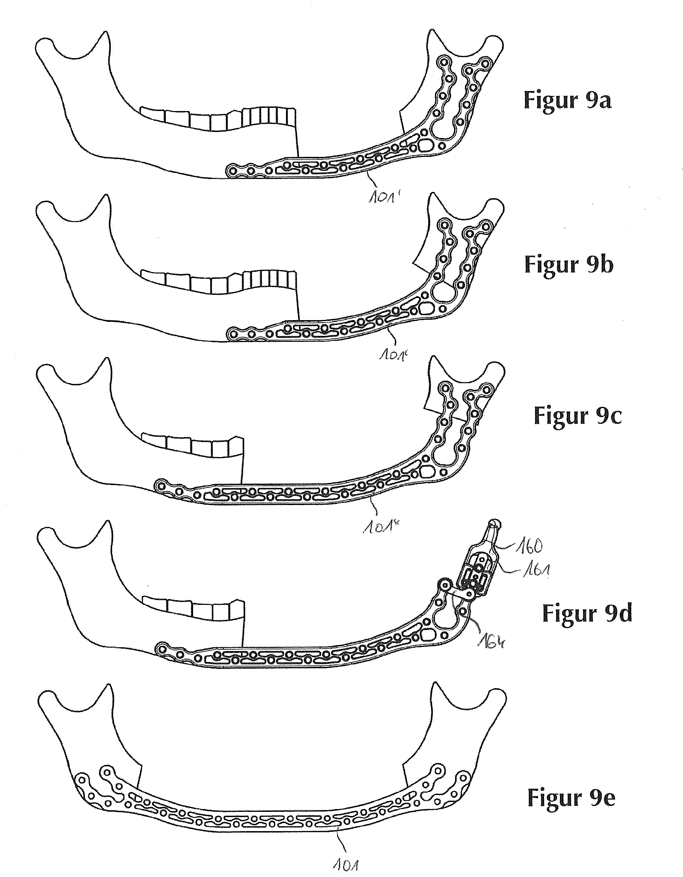

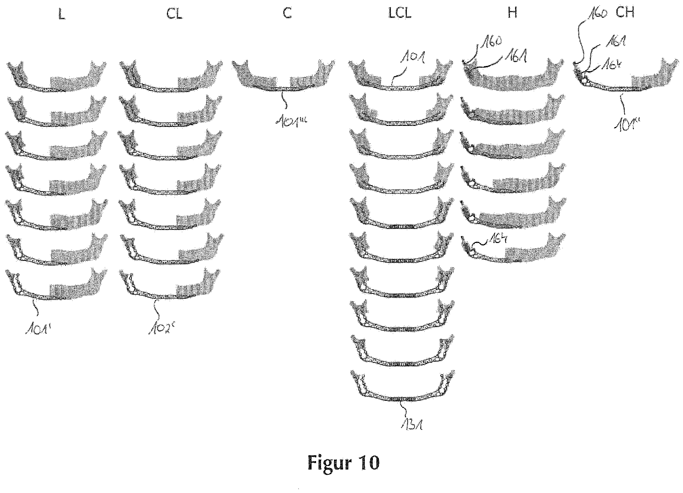

Ein solches Rekonstruktionsset benötigt nur eine vergleichsweise geringe Anzahl verschiedener Elemente, mit denen aber trotzdem ein Grossteil der Defekte menschlicher Unterkiefer behandelt werden kann, wie unten im Zusammenhang mit den Figuren 9a-i und 10 noch erläutert wird.

Optional kann das Rekonstruktionsset noch weitere, nicht erfindungsgemässe Knochenplatten enthalten - also beispielsweise Knochenplatten mit einem Hauptabschnitt, von dessen Enden sich jeweils nur ein Flügel oder gar kein Flügel erstreckt.

Die genannte Verbindungsplatte kann in wenigstens einem Endbereich zwei Öffnungen aufweisen, welche gleichzeitig mit mindestens zwei korrespondierenden Öffnungen mindestens einer der Knochenplatten oder mindestens eines Teils einer der Knochenplatten zur Deckung bringbar sind. Ein als Verbindungsschraube ausgebildetes Befestigungselement kann zur Verbindung eine der Öffnungen einer Knochenplatte durchdringen, und ein Aussengewinde der Verbindungsschraube kann in ein in der Verbindungsplatte befindliches Innengewinde eindringen.

Im Folgenden wird die Erfindung an Hand von mehreren Ausführungsbeispielen im Detail erläutert. Dabei zeigen

Figur 1: eine Panorama-Röntgen-Ansicht auf einen menschlichen Unterkiefer mit einer daran befestigten ersten erfindungsgemässen Knochenplatte;

Figur 2: eine Panorama-Röntgen-Ansicht auf einen menschlichen Unterkiefer mit einer daran befestigten zweiten erfindungsgemässen Knochenplatte;

Figur 3a: eine Panorama-Röntgen-Ansicht auf einen menschlichen Unterkiefer mit einer daran be- festigten dritten erfindungsgemässen Knochenplatte;

Figur 3b: eine perspektivische Schnittansicht entlang der in Figur 3a eingezeichneten Schnittlinie III-III;

Figur 4a: eine perspektivische Ansicht der zweiten erfindungsgemässen Knochenplatte gemäss Figur 2;

Figur 4b: eine perspektivische Detailansicht der zweiten erfindungsgemässen Knochenplatte;

Figur 5a: eine Draufsicht der zweiten erfindungsgemässen Knochenplatte;

| | |
|---|---|
| Figur 5b: | eine Schnittansicht der zweiten erfindungsgemässen Knochenplatte entlang der in Figur 5a dargestellten Schnittlinie E-E; |
| Figur 6a: | eine perspektivische Ansicht einer ersten Knochenschraube eines erfindungsgemässen chirurgischen Sets; |
| Figur 6b: | eine Draufsicht der ersten Knochenschraube eines erfindungsgemässen chirurgischen Sets; |
| Figur 7: | ein erstes Rekonstruktionsset mit einer ersten erfindungsgemässen Knochenplatte, einer zweiten erfindungsgemässen Knochenplatte und einer Verbindungsplatte in zwei Ansichten; |
| Figur 8: | ein zweites Rekonstruktionsset mit drei erfindungsgemässen Knochenplatten, einer nicht erfindungsgemässen Kno- chenplatte, einer Verbindungsplatte, einer rechten und einer linken Kiefergelenkprothese, einem Trägerelement zum Halten einer der Kiefergelenkprothesen, vier Verbindungsschrauben zum Verbinden der Kiefergelenkprothesen mit dem Trägerelement, vier Verbindungsschrauben zum wahlweisen Verbinden einer der Knochenplatten mit dem Trägerelement oder mit der Verbindungsplatte und einem Kiefergelenk-Stabilisierungselement; |
| Figuren 9a-i: | neun Panorama-Röntgen-Ansichten menschlicher Unterkiefer mit diversen Defekten und Elementen des Rekonstruktionssets gemäss Figur 8; |
| Figur 10: | eine weitere Übersicht über menschliche Unterkiefer mit diversen Defekten und Elementen des Rekonstruktionssets gemäss Figur 8; |
| Figuren 11a und b: | zwei Ansichten einer zweiten Knochenschraube mit einem Schraubenkopf und einem an der Spitze angeordneten Verblockungselement; |
| Figuren 12a und b: | zwei Ansichten einer dritten Knochenschraube ohne Schraubenkopf, aber mit einem an der Spitze angeordneten Verblockungselement; |
| Figuren 13a bis c: | drei perspektivische Ansichten eines erfindungsgemässen chirurgischen Sets mit einer erfindungsgemässen Knochenplatte und vier Knochenschrauben; |
| Figur 14a: | eine perspektivische Ansicht einer erfindungsgemässen Knochenplatte ohne Knochenschrauben; |
| Figur 14b: | eine perspektivische Ansicht der erfindungsgemässen Knochenplatte gemäss Figur 14a mit vier darin eingesetzten Knochenschrauben; |
| Figuren 15a bis c: | drei Detailansichten einer weiteren Knochenplatte mit einer Öffnung mit zwei Aufnahmebereichen; |
| Figuren 16a bis c: | drei Detailansichten noch einer weiteren Knochenplatte mit einer Öffnung mit zwei Aufnahmebereichen; |
| Figuren 17a bis c: | drei Detailansichten noch einer weiteren Knochenplatte mit einer Öffnung mit zwei Aufnahmebereichen; |
| Figuren 18a bis c: | drei Detailansichten noch einer weiteren Knochenplatte mit einer Öffnung mit zwei Aufnahmebereichen. |

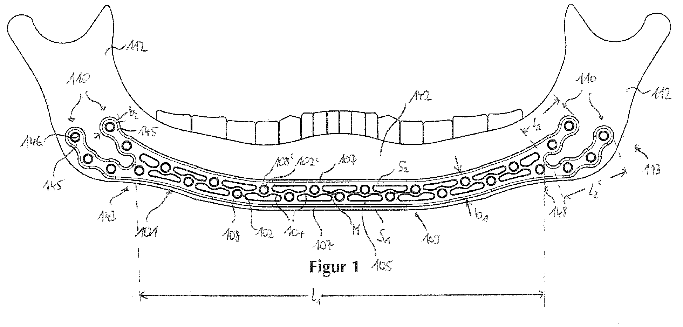

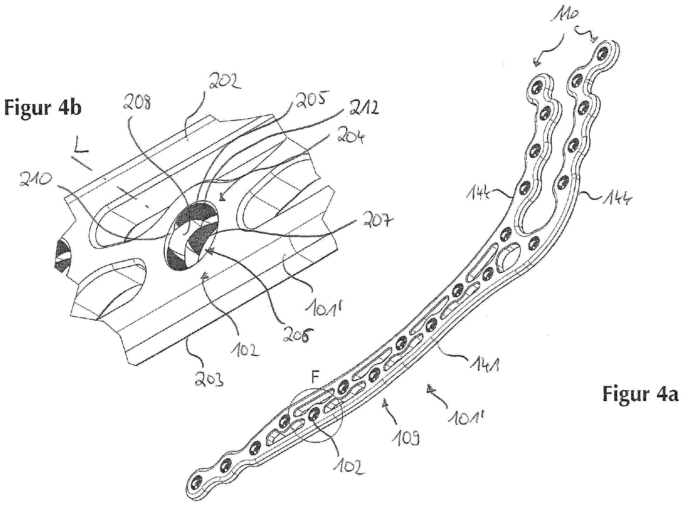

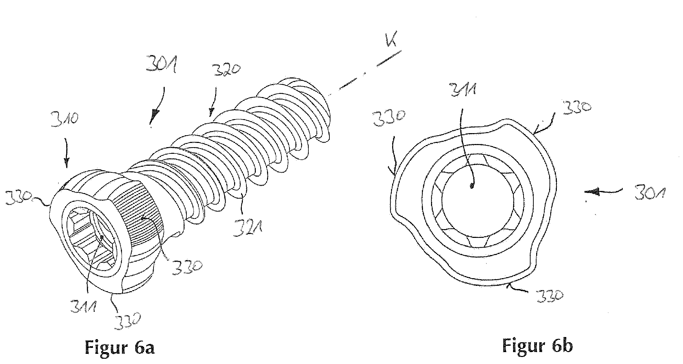

Figur 1 zeigt einen menschlichen Unterkiefer 113 mit einem Corpus 142 und zwei aufsteigenden Ästen 112. Zur klareren Darstellung wurde eine Projektion ähnlich einer zahnärztlichen Panorama-Röntgen-Ansicht gewählt, bei der die Aussenfläche des Unterkiefers 113 auf die Zeichenebene abgewälzt wurde. Am Unterkiefer 113 ist eine erste erfindungsgemässe Knochenplatte 101 angelegt und befestigt. Die Knochenplatte 101 enthält einen Hauptabschnitt 109, welcher über ein erstes Ende 143 und ein gegenüberliegendes zweites Ende 148 verfügt und sich entlang einer Mittellinie M vom ersten Ende 143 zum zweiten Ende 148 erstreckt. Der Hauptabschnitt 109 verfügt ferner über eine hier nicht erkennbare erste Anlegefläche 141, an der der Hauptabschnitt 109 am Corpus 142 angelegt und befestigt ist (siehe dazu Figur 4a). Zudem verfügt der Hauptabschnitt 109 über eine Mehrzahl von Aufnahmen 108, 108' mit jeweils einer kreisförmigen Öffnung 102, 102' zur Aufnahme jeweils einer hier nicht dargestellten Knochenschraube 301 (siehe dazu Figuren 6a und 6b).

Sowohl vom ersten Ende 143 als auch vom zweiten Ende 148 des Hauptabschnitts 109 erstrecken sich jeweils zwei nebeneinander angeordnete Flügel 110. Diese verfügen jeweils über eine hier nicht erkennbare zweite Anlegefläche 144 (siehe dazu ebenso Figur 4a) zum Anlegen und Befestigen an einer Aussenseite eines der aufsteigenden Äste 112. Ferner verfügen die Flügel 110 über Aufnahmen 145 mit jeweils einer kreisförmigen Öffnung 146 zur Aufnahme jeweils einer hier nicht dargestellten Knochenschraube 301 (siehe dazu Figuren 6a und 6b).

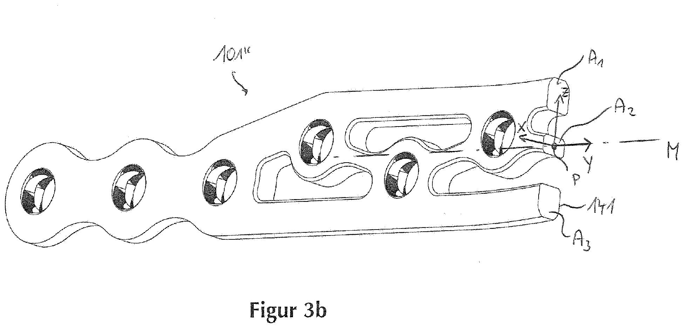

Der Hauptabschnitt 109 weist in einer hier nicht dargestellten Querschnittsebene bezüglich einer senkrecht zur ersten Anlegefläche 141 (und damit auch senkrecht zur Zeichenebene) verlaufenden Achse eine erste minimale Biegesteifigkeit auf (siehe Figur 3b für die Darstellung einer Querschnittsebene einer anderen Knochenplatte). Jeder Flügel 110 weist in hier ebenfalls nicht dargestellten jeweiligen Querschnittsebenen bezüglich senkrecht zur jeweiligen zweiten Anlegefläche 144 (und damit auch senkrecht zur Zeichenebene) verlaufenden Achsen eine jeweilige zweite minimale Biegesteifigkeit auf. Dabei ist die erste minimale Biegesteifigkeit grösser als jede der zweiten minimalen Biegesteifigkeiten, jedoch kleiner als die minimale Gesamtbiegesteifigkeit aller sich vom ersten Ende 143 erstreckenden Flügel 110 und auch kleiner als die minimale Gesamtbiegesteifigkeit aller sich vom zweiten Ende 148 erstreckenden Flügel 110.

Der Hauptabschnitt 109 weist eine Fachwerkstruktur auf. Er verfügt nämlich über Verstrebungen 104, welche transversal zur Mittellinie M verlaufen. Die Öffnungen 102 sind auf einer ersten Seite der Mittellinie M angeordnet, und die Öffnungen 102' sind auf einer der ersten Seite gegenüberliegenden Seite der Mittellinie M angeordnet. An den Knotenpunkten der Fachwerkstruktur sind hier die Aufnahmen 108, 108' mit den kreisförmigen Öffnungen 102, 102' angeordnet. Alternativ sind allerdings auch Knotenpunkte ohne Schraubenlöcher denkbar. Die Flügel 110 weisen im hier dargestellten Ausführungsbeispiel keine derartige Fachwerkstruktur auf.

Aus den genannten Biegeeigenschaften und der Fachwerkstruktur folgen die bereits oben im Detail erläuterten Vorteile der erfindungsgemässen Knochenplatte 101:

Im Ausgangszustand, insbesondere im Auslieferungszustand, kann die Knochenplatte 101 im Wesentlichen eben sein, was die Herstellung, den Transport und die Lagerung erleichtert. Um die Knochenplatte 101 an die individuelle Anatomie eines Patienten anzuformen, können durch geeignet gewählte Biegemomente innerhalb der Plattenebene im Wesentlichen nur die Flügel 110, nicht aber auch der Hauptabschnitt 109 verbogen werden. Hierdurch können im Wesentlichen nur die Flügel 110 in der Plattenebene an die individuelle Anatomie eines Patienten angeformt werden, beispielsweise an den Winkel zwischen Corpus 142 und aufsteigendem Ast 112. Der Hauptabschnitt 109 kann im Wesentlichen nur durch ein Biegen aus der durch die Anlegefläche 141 definierten Ebene heraus in eine anatomische Form deformiert werden, in welcher er am Unterkiefer 113 befestigt werden kann.

Wie sich in Studien gezeigt hat, ist bei geeigneter Formgebung der Knochenplatten eine Deformation des Hauptabschnitts 109 innerhalb der Plattenebene auch gar nicht erforderlich, um die Knochenplatte 101 an den Corpus 142 einer Vielzahl von menschlichen Unterkiefern 113 anformen zu können. Stattdessen hat die geringe Deformierbarkeit des Hauptabschnitts 109 innerhalb der Plattenebene den Vorteil, dass die Knochenplatte 101 gegenüber in dieser Plattenebene wirkenden Kräften und Biegemomenten stabil ist.

Ist die Knochenplatte 101 dann am Unterkiefer 113 befestigt, so sind die beiden Flügel 110 auch relativ zueinander fixiert, so dass in diesem implantierten Zustand die minimale Gesamtbiegesteifigkeit dieser Flügel 110 relevant ist. Da diese Gesamtbiegesteifigkeit grösser ist als die erste minimale Biegesteifigkeit des Hauptabschnitts 109, können im implantierten Zustand auch die Flügel 110 kaum noch innerhalb der Plattenebene deformiert werden, was auch in diesem Bereich zu einer höheren Stabilität gegenüber in der Plattenebene wirkenden Kräften und Biegemomenten führt. Ist der zu behandelnde Knochen ein Unterkiefer, so ist die Forderung, dass die Gesamtbiegesteifigkeit grösser ist als die erste Biegesteifigkeit, der realen Kraftverteilung im Unterkiefer geschuldet: Gelenksnah ist die Belastung auf den Knochen beim Kauen/Beissen grösser als zum Kinn hin.

Der Hauptabschnitt 109 ist an beiden Seiten $S_1$, $S_2$ von einer Rahmenstruktur 105 begrenzt, welche einen äusseren Rand 107 aufweist, der im Wesentlichen geradlinig verläuft. Hierdurch können Kanten vermieden werden, die beispielsweise zu Verletzungen des Körpergewebes führen könnten.

Der Hauptabschnitt 109 hat eine Breite $b_1$ von 10 mm und eine entlang der Mittellinie M gemessene Länge $l_1$ von 145 mm. Die Flügel 110 haben eine Breite $b_2$ von 7 mm. Die in Figur 1 oben dargestellten kürzeren Flügel 110 haben eine Länge $l_2$ von 19 mm, und die in Figur 1 unten dargestellten längeren Flügel 110 haben eine Länge $l_2'$ von 26 mm. Somit weist jeder Flügel 110 eine Breite $b_2$ auf, die höchstens 80% der Breite $b_1$ des Hauptabschnitts 109 beträgt.

Die Knochenplatte besteht aus einem biokompatiblen Implantatwerkstoff, wie beispielsweise Titan und seinen Legierungen, Implantatstahl, implantierbarem Kunststoff oder implantierbarer Keramik.

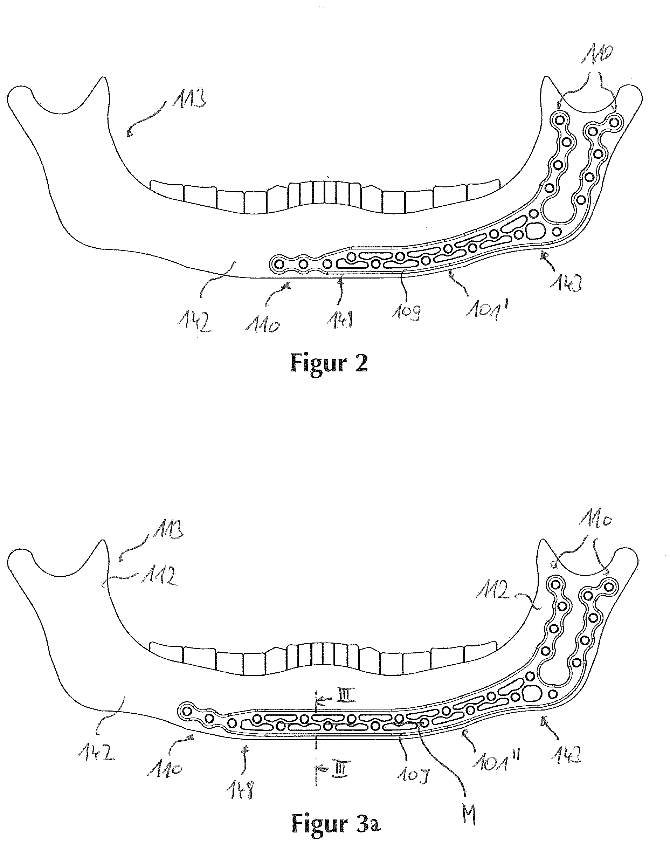

Die Figuren 2 und 3a zeigen einen menschlichen Unterkiefer 113 mit zwei weiteren erfindungsgemässen Knochenplatten 101' bzw. 101". Deren Hauptabschnitte 109 weisen jeweils nur an einem ersten Ende 143 zwei Flügel 110 auf. Am gegenüberliegenden zweiten Ende 148 ist jeweils nur ein einziger Flügel 110 angeordnet. Der Hauptabschnitt 109 der in Figur 3a dargestellten dritten erfindungsgemässen Knochenplatte 101'' ist länger als der Hauptabschnitt 109 der in der Figur 2 dargestellten zweiten erfindungsgemässen Knochenplatte 101'. Somit kann die in Figur 3a dargestellte dritte erfindungsgemässe Knochenplatte 101 einen grösseren Bereich des Corpus 142 abdecken, wie unten noch im Zusammenhang mit den Figuren 9a bis 9i und 10 klar wird.

In Figur 3b ist eine Querschnittsansicht entlang der Schnittlinie III-III in Figur 3a gezeigt. Die Querschnittsebene verläuft senkrecht zur Mittellinie M des Hauptabschnitts 109. Sie ist aber nicht notwendigerweise diejenige, an der die minimale Biegesteifigkeit des Hauptabschnitts 109 vorliegt. Eingezeichnet ist ein kartesisches Koordinatensystem, dessen Ursprung durch den gemeinsamen Flächenschwerpunkt P der drei partiellen Querschnittsflächen $A_1$, $A_2$ und $A_3$ gebildet wird. Die x-Achse steht senkrecht zur Anlegefläche 141, und die y-Achse verläuft im Wesentlichen parallel zur Mittellinie M. Die x-Achse und die z-Achse spannen zusammen die partiellen Querschnittsflächen $A_1$, $A_2$ und $A_3$ auf. Rechnerisch lässt sich das axiale Flächenträgheitsmoment durch das Integral

$$\int_A z^2 dA = \int_{A_1} z^2 dA + \int_{A_2} z^2 dA + \int_{A_3} z^2 dA$$

der quadrierten z-Koordinate über die aus den drei partiellen Querschnittsflächen $A_2$, $A_2$ und $A_3$ zusammengesetzte Querschnittsfläche A bestimmen. Für jedes der drei Integrale wir dabei ein gemeinsames Koordinatensystem verwendet, dessen Koordinatenursprung im Schwerpunkt der Gesamtfläche und nicht den Flächenschwerpunkten der partiellen Querschnittsflächen liegt. Die Biegesteifigkeit ergibt sich als Produkt dieses axialen Flächenträgheitsmomentes mit dem Elastizitätsmodul der Knochenplatte 101''. Wie oben erläutert wurde, ist es jedoch zur Feststellung, ob die Knochenplatte 101" die oben erläuterten Biegeeigenschaften erfüllt, nicht zwingend erforderlich, die erste minimale Biegesteifigkeit des Hauptabschnittes 109, die zweiten minimalen Biegesteifigkeiten der Flügel 110 und die minimale Gesamtbiegesteifigkeit der Flügel 110 nummerisch exakt zu kennen.

Figur 4a zeigt eine perspektivische Ansicht der zweiten erfindungsgemässen Knochenplatte 101' gemäss Figur 2. Der Hauptabschnitt 109 verfügt über eine erste Anlegefläche 141 zum Anlegen und Befestigen am Corpus 142, und die beiden Flügel 110 enthalten jeweils eine zweite Anlegefläche 144 zum Anlegen und Befestigen am aufsteigenden Ast 112.

Jede der Öffnungen 102, 102' und 146 verfügt über eine Struktur, die in der Detailansicht gemäss Figur 4b zu erkennen ist. Die Öffnung 102 dient der Aufnahme einer in den Figuren 6a und 6b dargestellten ersten Knochenschraube 301. Die Öffnung 102 durchdringt die Knochenplatte 101 entlang einer Längsachse L von einer Oberseite 202 der Knochenplatte 101 zu einer gegenüberliegenden Unterseite 203 der Knochenplatte 101. An der Oberseite 202 mündet die Öffnung 102 in einen ersten Aufnahmebereich 204, der von einer ersten Innenwand 205 begrenzt ist. An der Unterseite 203 mündet die Öffnung 102 in einen zweiten Aufnahmebereich 206, der von einer zweiten Innenwand 207 begrenzt ist.

In der ersten Innenwand 205 sind in Umfangsrichtung drei Ausnehmungen 208 gebildet. In jeder dieser drei Ausnehmungen 208 vergrössert sich der Abstand der ersten Innenwand 205 in Abhängigkeit vom Umlaufwinkel um die Längsachse L. Zudem ist im hier dargestellten Ausführungsbeispiel die erste Innenwand 205 im Bereich jeder der Ausnehmungen 208 sphärisch ausgebildet. Alternativ kann die erste Innenwand 205 aber beispielsweise auch paraboloidisch, ellipsoidisch oder hyperboloidisch ausgebildet sein. Analog sind in der zweiten Innenwand 207 in Umfangsrichtung drei Ausnehmungen 209 gebildet. In jeder dieser drei Ausnehmungen 209 vergrössert sich der Abstand der zweiten Innenwand 207 in Abhängigkeit vom Umlaufwinkel um die Längsachse L. Zudem ist im hier dargestellten Ausführungsbeispiel die zweite Innenwand 207 im Bereich jeder der Ausnehmungen 209 sphärisch ausgebildet, könnte aber alternativ ebenso paraboloidisch, ellipsoidisch oder hyperboloidisch ausgebildet sein. Der erste Aufnahmebereich 204 enthält ebenfalls eine sphärische Ansenkung 210 zur Aufnahme eines Verbindungselementes, insbesondere eines Schraubenkopfes, einer hier nicht dargestellten Knochenschraube. Ferner verfügt die erste Ausnehmung 204 über eine Auslaufkontur 212, welche zum Ausleiten einer Knochenschraube dient.

Die beiden Aufnahmebereiche 204, 206 sind für eine gleichdrehende Verblockung ausgebildet. Genauer sind beide Aufnahmebereiche 204, 206 für eine rechtsdrehende Verblockung ausgebildet. Ein Verblockungselement einer Knochenschraube kann also sowohl in Blickrichtung von der Oberseite 202 zur Unterseite 203 durch Drehen der Knochenschraube im Uhrzeigersinn im ersten Aufnahmebereich 204 fixiert werden als auch in Blickrichtung von der Unterseite 203 zur Oberseite 202 durch Drehen der Knochenschraube im Uhrzeigersinn im zweiten Aufnahmebereich 206 fixiert werden.

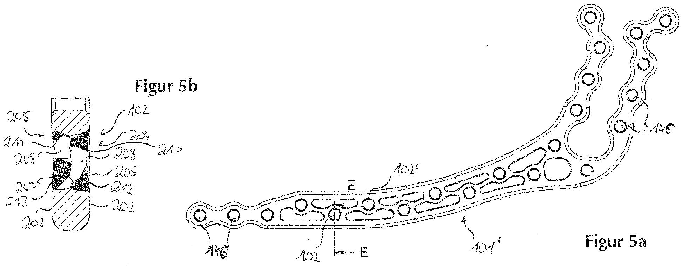

Figur 5a zeigt eine Draufsicht der zweiten erfindungsgemässen Knochenplatte 101'. Wie in der Schnittansicht gemäss Figur 5b zu erkennen ist, enthält der zweite Aufnahmebereich 206 ebenfalls eine sphärische Ansenkung 211 und eine Auslaufkontur 213.

Die Figuren 6a und 6b zeigen eine erste Knochenschraube 301, welche winkelvariabel in jeder der Öffnungen 102, 102' und 146 eingesetzt werden kann. Diese Knochenschraube 301 ist identisch zu der in WO 2004/086990 offenbarten. Sie weist einen Schraubenschaft 320 mit einem Gewinde 321 sowie einen als Verblockungselement ausgebildeten Schraubenkopf 310 auf, der über den Schraubenschaft 320 und das Gewinde 321 nach aussen vorsteht. Der Schraubenkopf 310 weist eine Eingriffskontur 311 auf, in die beispielsweise ein Schraubendreher eingesetzt werden kann, um die Knochenschraube 301 ein- oder auszudrehen. Ferner ist der Schraubenkopf 310 mit einer umlaufenden Aussenfläche versehen, die sich im Wesentlichen in Richtung einer Längsachse K der Knochenschraube 301 erstreckt und drei in Umfangsrichtung gleichmässig verteilte Klemmflächen 330 aufweist. In einer Azimutebene senkrecht zur Längsachse K betrachtet erweitern sich die Klemmflächen 330 keilförmig nach aussen und von der Längsachse K weg. Die Aussenfläche ist im Bereich der Klemmflächen 330 sphärisch ausgebildet. Diese Klemmflächen 330 erlauben es, den Schraubenkopf 310 wahlweise mit der ersten Innenwand 205 oder der zweiten Innenwand 207 verblocken zu können, so wie es im Detail in WO 2004/086990 beschrieben ist (welche jedoch lediglich eine Öffnung mit einem einzigen wie hier offenbarten Aufnahmebereich offenbart).

Auf diese Weise kann die Knochenschraube 301 sowohl von der Oberseite 202 in Richtung der Unterseite 203 durch die Öffnung 102 eingesetzt und so winkelvariabel an der Knochenplatte fixiert oder auch von der Unterseite 203 in Richtung der Oberseite 202 durch die Öffnung 102 eingesetzt und so winkelvariabel an der Knochenplatte fixiert werden. Damit können sowohl die Oberseite 202 als auch die Unterseite 203 wahlweise als Anlegeflächen dienen, die an den Knochen angelegt werden. Die Knochenplatten können daher wahlweise und je nach Bedarf für linksseitige oder für rechtsseitige Defekte eingesetzt werden, ohne dass auf eine winkelvariable Fixierung verzichtet werden muss. Dies reduziert deutlich das bereitzuhaltende Sortiment an Knochenplatten.

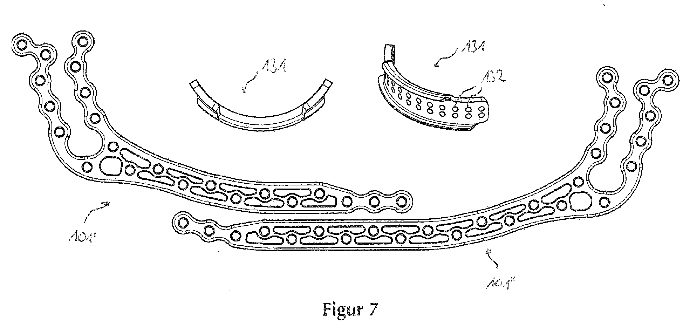

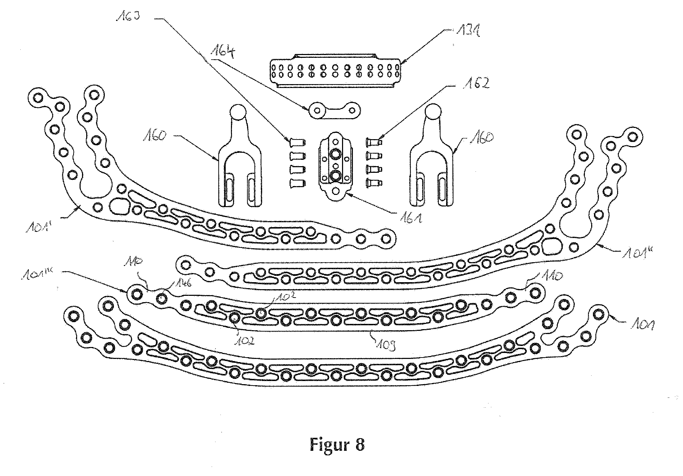

Das in Figur 7 dargestellte Rekonstruktionsset enthält die beiden in den Figuren 2 bis 3b dargestellten erfindungsgemässen Knochenplatten 101' und 101" sowie eine Verbindungsplatte 131, die hier doppelt in zwei verschiedenen Ansichten dargestellt ist. Als Mittel zum Verbinden mit einer Knochenplatte, beispielsweise einer der oben beschriebenen Knochenplatten, enthält die Verbindungsplatte 131 mehrere Öffnungen 132 zur Aufnahme eines Befestigungselementes, wie beispielsweise einer in Figur 8 dargestellten Verbindungsschraube 163. Die Verbindungsschraube 163 kann zur Verbindung eine der Öffnungen 102, 102' oder 146 der erfindungsgemässen Knochenplatten oder auch eine Öffnung einer anderen Knochenplatte durchdringen, und ihr Gewinde kann in ein in der Verbindungsplatte befindliches Gewinde eindringen. Dies erlaubt eine variable Verlängerung oder gar die Kopplung zweier Platten zur Anpassung an die individuelle Anatomie bzw. den individuellen Knochendefekt.

Figur 8 zeigt ein zweites Rekonstruktionsset. Dieses enthält

- die erfindungsgemässen Knochenplatten 101, 101' und 101" gemäss Figuren 1 bis 3b;

- eine nicht erfindungsgemässe Knochenplatte 101''', die an jedem Ende des Hauptabschnitts 109 jeweils nur über einen einzigen Flügel 110 verfügt;

- eine wie in Figur 7 wiedergegebene Verbindungsplatte 131;

- je eine Kiefergelenkprothese 160 für die linke und die rechte Seite;

- ein Trägerelement 161 zum Halten und höhenvariablen Einstellen einer der Kiefergelenkprothesen 160 und zur Befestigung an einer Knochenplatte oder zur direkten Befestigung am Knochen (alternativ kann das Rekonstruktionsset auch mehrere Trägerelemente 161 enthalten);

- Verbindungsschrauben 162 (im hier dargestellten Ausführungsbeispiel vier Stück) zum Verbinden einer der Kiefergelenkprothesen 160 mit dem Trägerelement 161;

- Verbindungsschrauben 163 (im hier dargestellten Ausführungsbeispiel vier Stück) zum Verbinden des Trägerelements 161 mit einem der Flügel 110 oder zum Verbinden der Verbindungsplatte 131 mit einer der Knochenplatten;

- ein Kiefergelenk-Stabilisierungselement 164 zur Querverbindung zweier Flügel 110 mittels Verbindungsschrauben 163 (alternativ kann das Rekonstruktionsset auch mehrere Kiefergelenk-Stabilisierungselemente 164 enthalten).

In den Figuren 9a bis 9i ist dargestellt, wie das Rekonstruktionsset gemäss Figur 8 für eine Vielzahl von Knochendefekten eingesetzt werden kann:

- Gemäss Figuren 9a und 9b können mit Hilfe der kürzeren Knochenplatte 101' kleinere Knochendefekte behandelt werden.

- Figur 9c zeigt, wie mittels der längeren Knochenplatte 101" ein grösserer Defekt behandelt werden kann.

- Mit Hilfe einer gekürzten Knochenplatte 101", des Trägerelements 161, einer Kiefergelenkprothese 160 und eines Kiefergelenk-Stabilisierungselements 164 sowie der zugehörigen Verbindungsschrauben 162 und 163 kann auch ein Kiefergelenk ersetzt werden, wie in Figur 9d dargestellt ist.

- Figur 9e zeigt die Verwendung der Knochenplatte 101 für einen grossen zentralen Defekt.

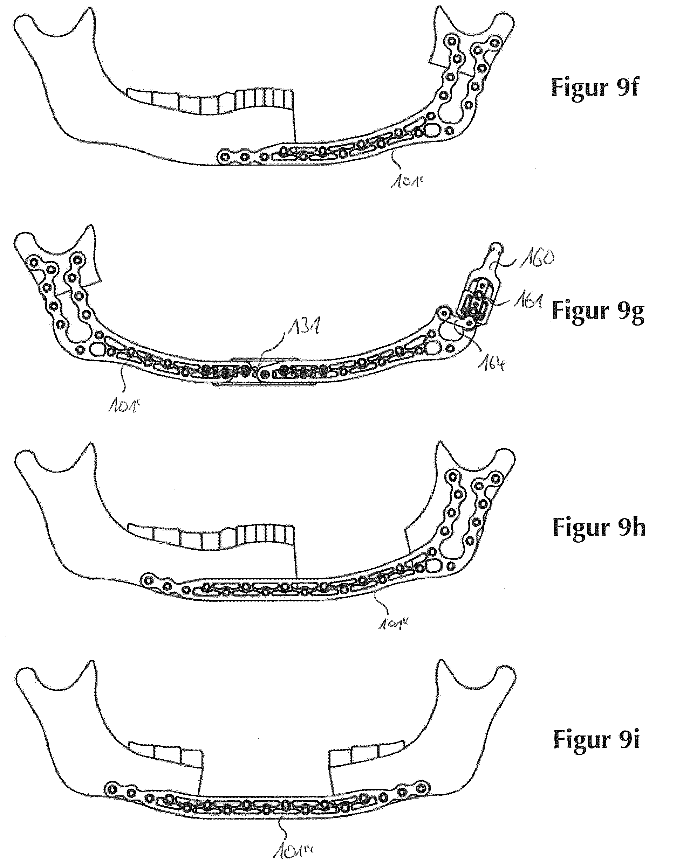

- In Figur 9f ist nochmals eine Verwendung der Knochenplatte 101' dargestellt, bei der der Defekt noch grösser ist als in den Figuren 9a und 9b.

- In der Situation gemäss Figur 9g wurden zwei Knochenplatten 101' und 101" verwendet, die mit Hilfe der Verbindungsplatte 131 verbunden wurden. Während die Knochenplatte 101' im Bereich des Kinns zurechtgeschnitten wurde, erfolgte dies bei der Knochenplatte 101'' im Bereich des Kinns und des aufsteigenden Asts. Zudem wurden hier ebenfalls eine Kiefergelenkprothese 160, das Trägerelement 161 und das Kiefergelenk-Stabilisierungselement 164 mit zugehörigen Verbindungsschrauben 162 und 163 eingesetzt.

- Figur 9h zeigt nochmals eine Verwendung der längeren Knochenplatte 101''.

- In Figur 9i ist schliesslich der Einsatz der Knochenplatte 101''' zu erkennen.

Alternativ ist aber auch ein Rekonstruktionsset denkbar, bei dem eine Kiefergelenkprothese direkt, also ohne zusätzliches Trägerelement, mit einer der Knochenplatten verbunden werden kann. Figur 10 zeigt nochmals eine Übersicht über eine Vielzahl von Knochendefekten gemäss der üblichen HCL-Klassifikation:

- Die L-Defekte (laterale Kontinuitätsdefekte) können mit der kürzeren Knochenplatte 101' behandelt werden, die nur an einem Ende über zwei Flügel verfügt. Dargestellt sind nur Defekte auf der rechten Patientenseite, aufgrund der oben beschriebenen doppelten Aufnahmebereiche der Öffnungen ist die analoge Anwendung derselben Knochenplatte 101' auf der linken Patientenseite auch möglich.

- Für CL-Defekte (laterale und zentrale Kontinuitätsdefekte) kann die längere Knochenplatte 102' eingesetzt werden, die ebenfalls nur an einem Ende zwei Flügel enthält. Dargestellt sind nur Defekte auf der rechten Patientenseite, aufgrund der oben beschriebenen beidseitigen Verblockung ist die analoge Anwendung derselben Knochenplatte 102' auf der linken Patientenseite auch möglich.

- C-Defekte (zentrale Kontinuitätsdefekte) können mit Hilfe der Knochenplatte 101''' behandelt werden, die in der hier gezeigten Ausführung an keiner der beiden Enden über zwei Flügel verfügt. Platten für C-Defekte mit ein- oder beidseitig zwei Flügeln sind jedoch denkbar.

- Für die Behandlung von LCL-Defekten (doppelt laterale und zentrale Kontinuitätsdefekte) kann die Knochenplatte 101 (obere vier Darstellungen) oder die Knochenplatten 101' oder 101" zusammen mit der Verbindungsplatte 131 (untere sechs Darstellungen) verwendet werden.

- Für H-Defekte (hemimandibuläre Kontinuitätsdefekte) kann entweder nur eine Kiefergelenkprothese 160 mit Trägerelement 161 eingesetzt werden oder zusätzlich das Kiefergelenk-Stabilisierungselement 164 und eine der Knochenplatten. Bei der in der Spalte "H" ganz oben dargestellten Anwendung wird keine Knochenplatte eingesetzt; stattdessen wird das Trägerelement 161 direkt am Knochen befestigt. Auch hier ist nur die Verwendung auf der rechten Patientenseite dargestellt (s.o.).

- Für CH-Defekte (hemimandibuläre und zentrale Kontinuitätsdefekte) kann eine Kombination aus an den Flügeln 110 abgeschnittener Knochenplatte 101'', Kiefergelenk-Stabilisierungselement 164, Trägerelement 161 und Kiefergelenkprothese 160 verwendet werden. Auch hier ist nur die Verwendung auf der rechten Patientenseite dargestellt (s.o.).

Insgesamt zeigt es sich, dass sämtliche Knochendefekte mit einem vergleichsweise kleinen Rekonstruktionsset behandelt werden können. Dies ist unter anderem durch die beidseitige Verwendbarkeit der Knochenplatten bedingt, die auf die erfindungsgemässen doppelten Aufnahmebereiche der Öffnungen zurückzuführen ist.

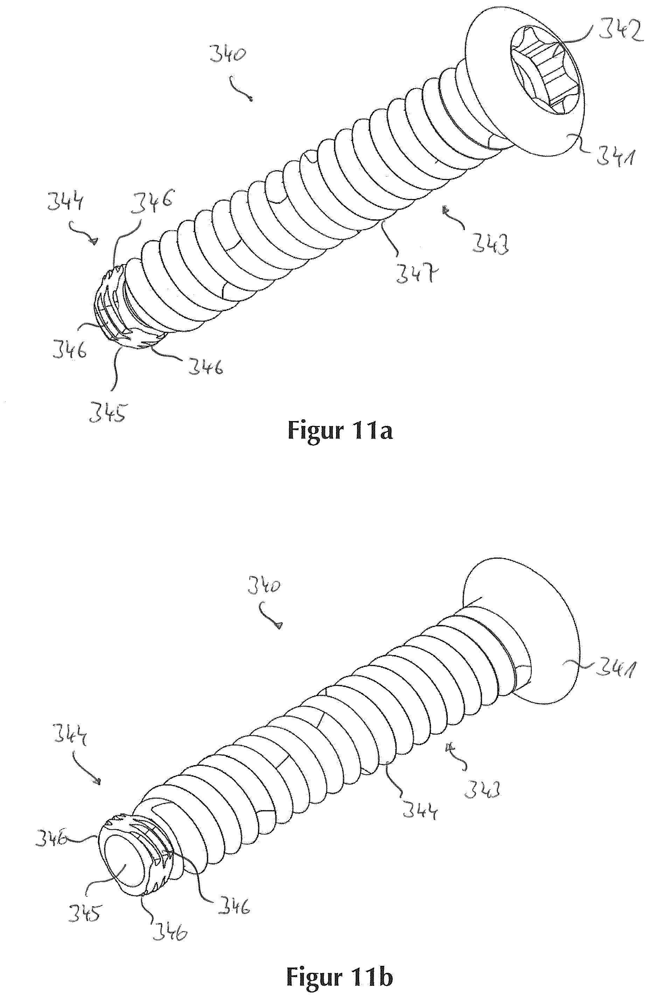

In den Figuren 11a und 11b ist eine zweite Knochenschraube 340 dargestellt. Diese enthält einen Schraubenkopf 341 mit einer Eingriffskontur 342, einen Schraubenschaft 343 mit einem Gewinde 347 sowie ein an einem dem Schraubenkopf 341 gegenüberliegenden Ende 344 der Knochenschraube 340 angeordnetes Verblockungselement 345. Dieses Verblockungselement 345 enthält drei in Umfangsrichtung gleichmässig verteilte Klemmflächen 346, die ausgebildet sind wie in WO 2004/086990 offenbart.

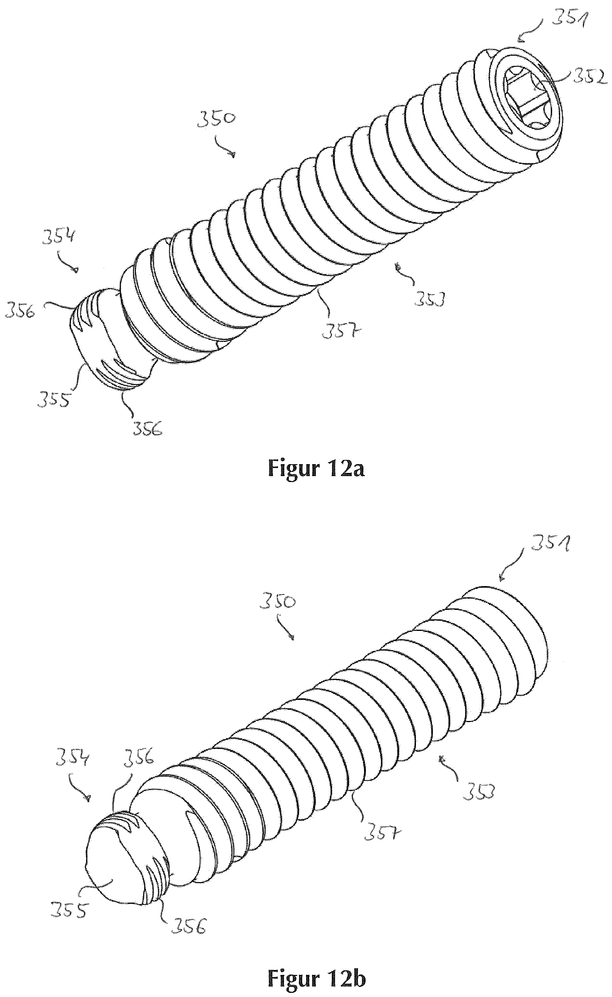

Die Figuren 12a und 12b zeigen eine dritte Knochenschraube 350, die jedoch keinen Schraubenkopf aufweist. An einem ersten Ende 351 verfügt sie über eine Eingriffskontur 352, und an einem dem ersten Ende 351 gegenüberliegenden zweiten Ende 354 weist sie ein Verblockungselement 355 auf, welches ebenso wie das in den Figuren 11a und 11b dargestellte Verblockungselement 345 über drei Klemmflächen 356 verfügt. Zwischen dem ersten Ende 351 und dem zweiten Ende 354 erstreckt sich ein Schraubenschaft 353 mit einem Gewinde 357.

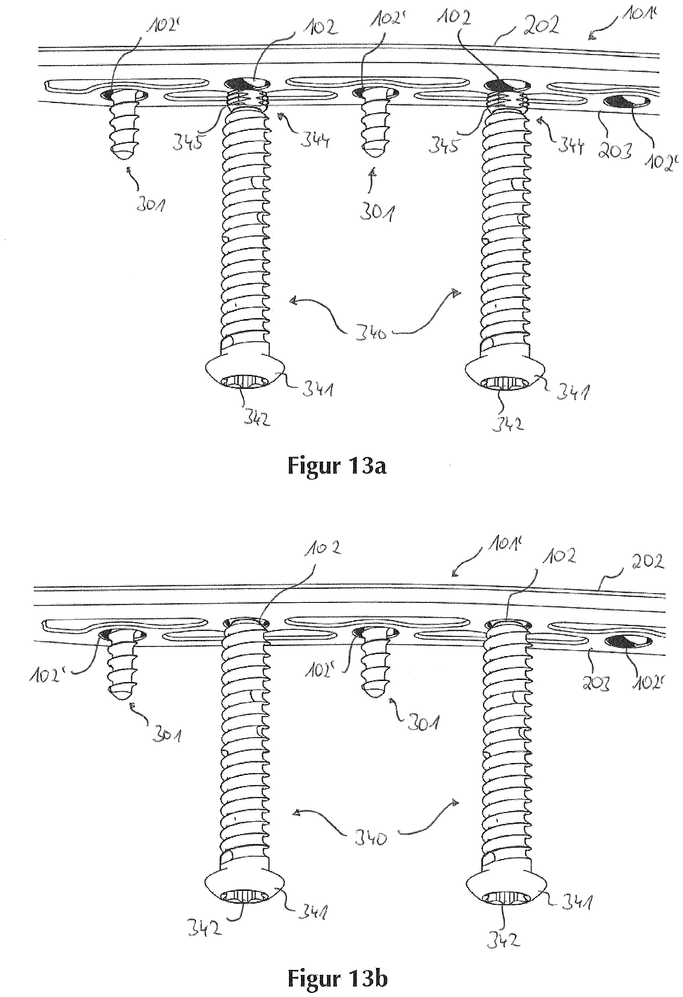

Die Figuren 13a bis 13c zeigen die erfindungsgemässe Knochenplatte 101' aus Figur 2 mit zwei wie in den Figuren 11a und 11b dargestellten Knochenschrauben 340 sowie zwei wie in den Figuren 6a und 6b gezeigte Knochenschrauben 301.

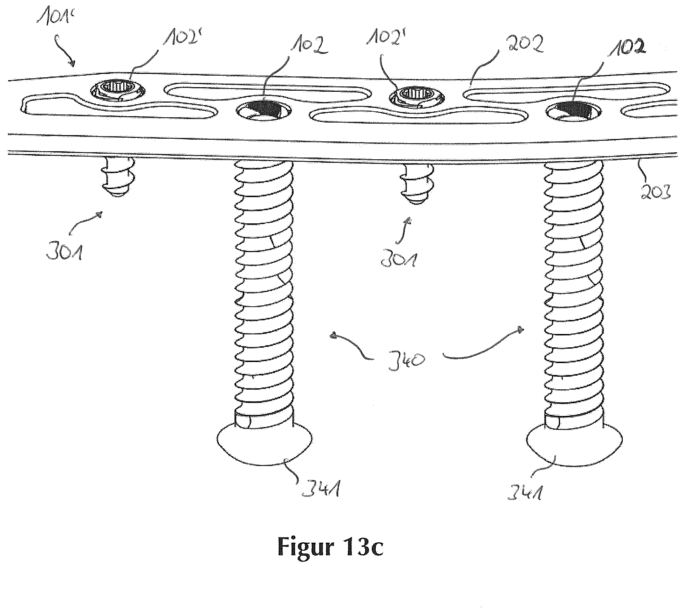

In der Ansicht gemäss Figur 13a sind die kürzeren Knochenschrauben 301 gemäss Figuren 6a und 6b von der Oberseite 202 der Knochenplatte 101' in Richtung der Unterseite 203 eingesetzt und durchdringen die Öffnungen 102'. Die Knochenschrauben 340 gemäss Figuren 11a und 11b sind mit ihren Enden 344 auf die Unterseite 203 hin gerichtet, jedoch noch nicht in Kontakt damit. In der Position gemäss Figur 13b sind die Knochenschrauben 340 mittels der Verblockungselemente 345 in den Öffnungen 102 eingesetzt und fixiert. Figur 13c enthält eine perspektivische Ansicht auf die Oberseite 202 der Knochenplatte 101'.

Falls mit den Knochenschrauben 301 alleine keine ausreichende Fixierung erreicht werden kann, so können die Knochenschrauben 340 für eine zusätzliche Stabilität sorgen. Beispielsweise können die kürzeren Knochenschrauben 301 durch die Knochenplatte 101' hindurch und dann von aussen in einen Unterkiefer eindringen, und die längeren Knochenschrauben 340 können den Unterkiefer vollständig von innen nach aussen durchdringen und dann in die Knochenplatte 101' eingreifen. Eine denkbare Indikation ist die Degradierung eines Knochens, die beispielsweise durch eine vorangehende Bestrahlung eingetreten sein kann.

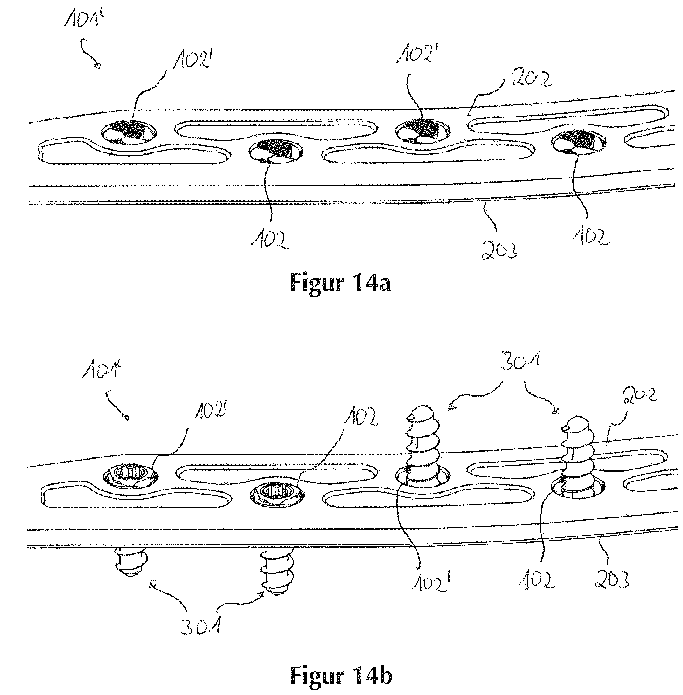

Die Figuren 14a und 14b zeigen die gleiche Knochenplatte 101', in deren Öffnungen 102, 102' jedoch gemäss Figur 14b vier der kürzeren Knochenschrauben 301 eingesetzt werden können. Dabei durchdringen eine erste Knochenschraube 301 und eine zweite Knochenschraube 301 die Knochenplatte 101' von der Oberseite 202 zur Unterseite 203, wobei die erste Knochenschrauben 301 eine Öffnung 102 und die zweite Knochenschraube 301 eine Öffnung 102' durchdringt. Eine dritte Knochenschraube 301 und eine vierte Knochenschraube 301 durchdringen die Knochenplatte 101' von der Unterseite 203 zur Oberseite 202, wobei die dritte Knochenschrauben 301 eine Öffnung 102 und die vierte Knochenschraube 301 eine Öffnung 102' durchdringt.

In einer möglichen Anwendung könnte ein erster Teil der Knochenplatte 101' im Bereich eines Kiefergelenks von der Mundhöhle aus verschraubt werden, während ein zweiter Teil der Knochenplatte 101' in der Corpusregion von aussen verschraubt werden könnte.

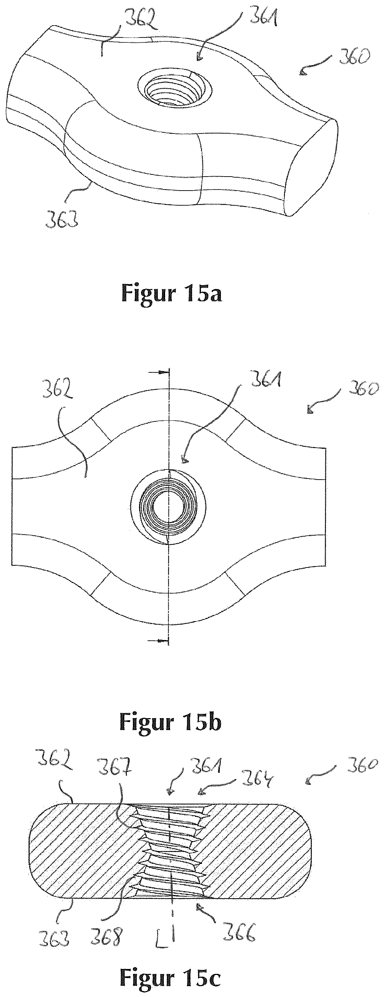

Die Figuren 15a bis 15c zeigen Ausschnitte einer weiteren Knochenplatte 360, die in Figur 15a in einer perspektivischen Ansicht, in Figur 15b in einer Draufsicht und in Figur 15c in einer Schnittansicht entlang der in Figur 15b eingezeichneten Schnittlinie dargestellt ist. Die Knochenplatte 360 enthält eine Öffnung 361, die an einer Oberseite 362 der Knochenplatte 360 in einen ersten Aufnahmebereich 364 mündet und an einer Unterseite 363 in einen zweiten Aufnahmebereich 366 mündet. Sowohl der erste Aufnahmebereich 364 als auch der zweite Aufnahmebereich 366 enthalten ein konusförmiges Innengewinde 367 bzw. 368, die sich in Richtung der Oberseite 362 bzw. der Unterseite 363 aufweiten. In diesem Ausführungsbeispiel sind beide Innengewinde 367, 368 zueinander identisch.

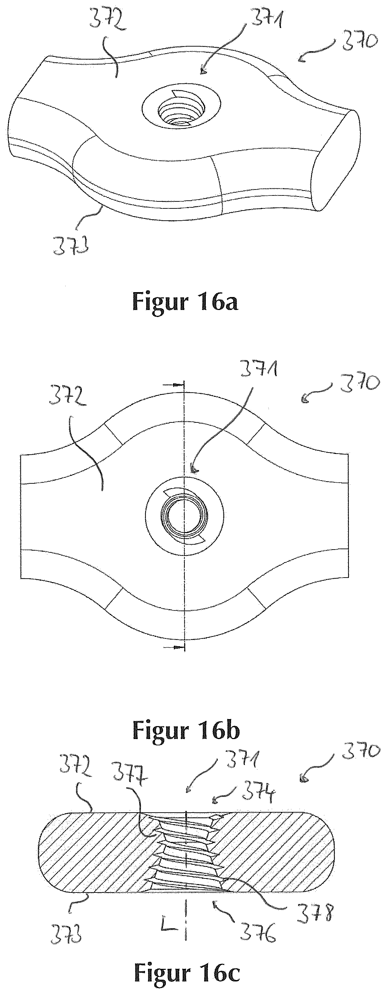

Die Figuren 16a bis 16c zeigen Ausschnitte einer weiteren Knochenplatte 370, die in Figur 16a in einer perspektivischen Ansicht, in Figur 16b in einer Draufsicht und in Figur 16c in einer Schnittansicht entlang der in Figur 16b eingezeichneten Schnittlinie dargestellt ist. Die Knochenplatte 370 enthält eine Öffnung 371, die an einer Oberseite 372 der Knochenplatte 370 in einen ersten Aufnahmebereich 374 mündet und an einer Unterseite 373 in einen zweiten Aufnahmebereich 376 mündet. Sowohl der erste Aufnahmebereich 374 als auch der zweite Aufnahmebereich 376 enthalten ein konusförmiges Innengewinde 377 bzw. 378, die sich in Richtung der Oberseite 372 bzw. der Unterseite 373 aufweiten. In diesem Ausführungsbeispiel sind nur die Öffnungswinkel der beiden Innengewinde 377, 378 identisch; das erste Innengewinde 377 ist jedoch höher als das zweite Innengewinde 378.

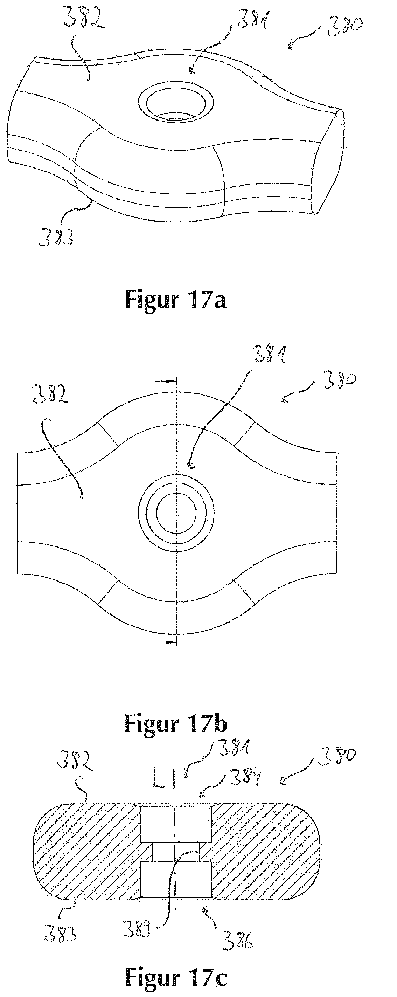

Die Figuren 17a bis 17c zeigen Ausschnitte einer weiteren Knochenplatte 380, die in Figur 17a in einer perspektivischen Ansicht, in Figur 17b in einer Draufsicht und in Figur 17c in einer Schnittansicht entlang der in Figur 17b eingezeichneten Schnittlinie dargestellt ist. Die Knochenplatte 380 enthält eine Öffnung 381, die an einer Oberseite 382 der Knochenplatte 380 in einen ersten Aufnahmebereich 384 mündet und an einer Unterseite 383 in einen zweiten Aufnahmebereich 386 mündet. Sowohl der erste Aufnahmebereich 384 als auch der zweite Aufnahmebereich 386 sind kreiszylinderförmig ausgebildet, enthalten aber keine Innengewinde. Zwischen den beiden Aufnahmebereichen 384, 386 ist ein ebenfalls kreiszylinderförmiger Zwischenbereich 389 vorhanden, dessen Radius jedoch kleiner ist als der der Aufnahmebereiche 384, 386.

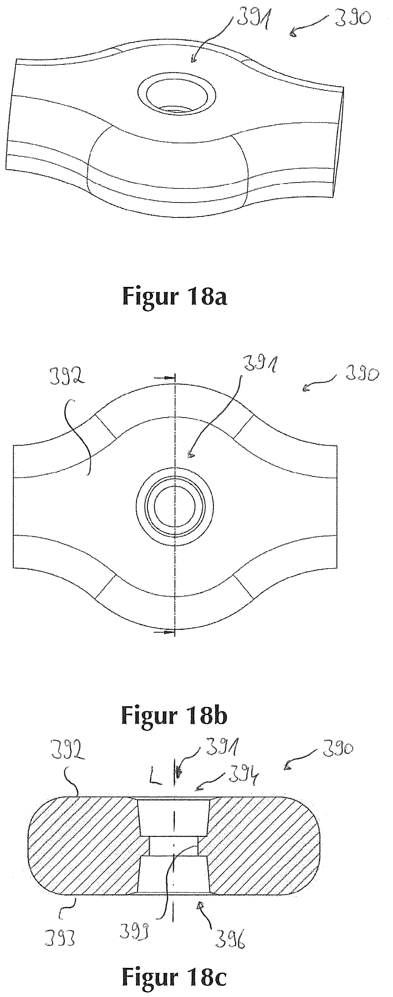

Die Figuren 18a bis 18c zeigen Ausschnitte einer weiteren Knochenplatte 390, die in Figur 18a in einer perspektivischen Ansicht, in Figur 18b in einer Draufsicht und in Figur 18c in einer Schnittansicht entlang der in Figur 18b eingezeichneten Schnittlinie dargestellt ist. Die Knochenplatte 390 enthält eine Öffnung 391, die an einer Oberseite 392 der Knochenplatte 390 in einen ersten Aufnahmebereich 394 mündet und an einer Unterseite 393 in einen zweiten Aufnahmebereich 396 mündet. Sowohl der erste Aufnahmebereich 394 als auch der zweite Aufnahmebereich 396 sind konusförmig ausgebildet und weiten sich in Richtung der Oberseite 392 bzw. der Unterseite 393 auf. Zwischen den beiden Aufnahmebereichen 394, 396 ist ein kreiszylinderförmiger Zwischenbereich 399 vorhanden, dessen Radius kleiner ist als der kleinste Radius der Aufnahmebereiche 394, 396.

In die Öffnungen 361, 371, 381, 391 der in den Figuren 15a bis 18c dargestellten Knochenplatten 360, 370, 380, 390 können Knochenschrauben mit geschlitztem Schraubenkopf (wie etwa in CH 669 105 A5) oder mit sphärischem Schraubenkopf (wie etwa in CH 675 531 A5) eingesetzt werden.

## Patentansprüche

**1.** Knochenplatte (101; 101'; 101''; 360; 370; 380; 390), enthaltend mindestens eine Öffnung (102, 102', 146; 361; 371; 381; 391) zur Aufnahme jeweils mindestens einer Knochenschraube (301; 340; 350),

wobei die Öffnung (102, 102', 146; 361; 371; 381; 391) die Knochenplatte (101; 101'; 101''; 360; 370; 380; 390) entlang einer Längsachse (L) von einer Oberseite (202; 362; 372; 382; 392) zu einer gegenüberliegenden Unterseite (203; 363; 373; 383; 393) durchdringt, wobei die Öffnung (102, 102', 146; 361; 371; 381; 391) an der Oberseite (202; 362; 372; 382; 392) in einen ersten Aufnahmebereich (204; 364; 374; 384; 394) mündet, der zur Aufnahme und Fixierung eines Verblockungselementes (310; 345; 355) einer Knochenschraube (301; 340; 350) in einer ersten Richtung ausgebildet ist, **dadurch gekennzeichnet, dass** die Öffnung (102, 102', 146; 361; 371; 381; 391) an der Unterseite (203; 363; 373; 383; 393) in einen zweiten Aufnahmebereich (206; 366; 376; 386; 396) mündet, der zur Aufnahme und Fixierung des Verblockungselementes (310; 345; 355) in einer zweiten Richtung ausgebildet ist, wobei die zweite Richtung im Wesentlichen entgegengesetzt zur ersten Richtung ist.

2. Knochenplatte (101; 101'; 101'') gemäss Anspruch 1, wobei der erste Aufnahmebereich (204) von einer ersten Innenwand (205) begrenzt ist der zweite Aufnahmebereich (206) von einer zweiten Innenwand (207) begrenzt ist, wobei sowohl in der ersten Innenwand (205) als auch in der zweiten Innenwand (207) jeweils mindestens eine Ausnehmung (208, 209) gebildet ist, wobei sich in jeder dieser Ausnehmungen (208, 209) der Abstand der jeweiligen Innenwand (205, 207) in Abhängigkeit vom Umlaufwinkel um die Längsachse (L) weg vergrössert.

3. Knochenplatte (101; 101'; 101'') gemäss Anspruch 2, wobei sowohl die erste Innenwand (205) als auch die zweite Innenwand (207) im Bereich jeder der jeweiligen Ausnehmungen (208, 209) zumindest annähernd sphärisch, paraboloidisch, ellipsoidisch oder hyperboloidisch ausgebildet ist.

4. Knochenplatte (101; 101'; 101'') gemäss einem der vorangehenden Ansprüche, wobei der erste Aufnahmebereich (204; 364; 374; 384; 394) zur winkelvariablen Fixierung des Verblockungselementes (310; 345; 355) in der ersten Richtung ausgebildet ist und der zweite Aufnahmebereich (206; 366; 376; 386; 396) zur winkelvariablen Fixierung des Verblockungselementes (310; 345; 355) in der zweiten Richtung ausgebildet ist.

5. Chirurgisches Set, enthaltend mindestens eine Knochenplatte (101; 101'; 101''; 360; 370; 380; 390) gemäss einem der vorangehenden Ansprüche sowie mindestens eine Knochenschraube (301; 340; 350) mit einem Schraubenschaft (320; 343; 353) und einem Verblockungselement (310; 345; 355), insbesondere einem über den Schraubenschaft (320) und ein Gewinde (321) des Schraubenschaftes (320; 343; 353) nach aussen vorstehenden Schraubenkopf (310), wobei das Verblockungselement (310; 345; 355) wahlweise im ersten Aufnahmebereich (204; 364; 374; 384; 394) oder im zweiten Aufnahmebereich (206; 366; 376; 386; 396) der Öffnung (102, 102', 146; 361; 371; 381; 391) aufnehmbar und insbesondere winkelvariabel fixierbar ist.

6. Chirurgisches Set gemäss Anspruch 5, wobei das Verblockungselement (310; 345; 355) mit einer umlaufenden Aussenfläche versehen ist, die sich im Wesentlichen in Richtung einer Längsachse (K) der Knochenschraube (301; 340; 350) erstreckt und mindestens eine Klemmfläche (330; 346; 356) aufweist, welche sich - in einer Azimutebene senkrecht zur Längsachse (K) betrachtet - keilförmig nach aussen von der Längsachse (K) weg erweitert.

7. Chirurgisches Set gemäss Anspruch 6, wobei die umlaufende Aussenfläche des Verblockungselementes (310; 345; 355) wenigstens im Bereich der Klemmfläche (330; 346; 356) zumindest annähernd sphärisch, paraboloidisch, ellipsoidisch oder hyperboloidisch ausgebildet ist.

**Claims**

1. Bone plate (101; 101'; 101''; 360; 370; 380; 390), containing at least one opening (102, 102', 146; 361; 371; 381; 391) for receiving at least one respective bone screw (301; 340; 350), wherein the opening (102, 102', 146; 361; 371; 381; 391) extends through the bone plate (101; 101'; 101''; 360; 370; 380; 390) along a longitudinal axis (L) from an upper side (202; 362; 372; 382; 392) to an opposite lower side (203; 363; 373; 383; 393), wherein the opening (102, 102', 146; 361; 371; 381; 391) at the upper side (202; 362; 372; 382; 392) opens into a first receiving region (204; 364; 374; 384; 394), which is designed to receive and fix a locking element (310; 345; 355) of a bone screw (301; 340; 350) in a first direction, **characterized in that** the opening (102, 102', 146; 361; 371; 381; 391) on the underside (203; 363; 373; 383; 393) opens into a second receiving region (206; 366; 376; 386; 396), which is designed to receive and fix the locking element (310; 345; 355) in a second direction, the second direction being essentially opposite to the first direction.

**2.** Bone plate (101; 101'; 101'') according to claim 1, wherein the first receiving region (204) is bounded by a first inner wall (205), the second receiving region (206) is bounded by a second inner wall (207), wherein at least one recess (208, 209) is formed in each of the first inner wall (205) and the second inner wall (207), wherein in each of these recesses (208, 209) the distance of the respective inner wall (205, 207) increases as a function of the angle of rotation about the longitudinal axis (L).

**3.** Bone plate (101; 101'; 101'') according to claim 2, wherein both the first inner wall (205) and the second inner wall (207) are at least approximately spherical, paraboloidal, ellipsoidal or hyperboloidal in the region of each of the respective recesses (208, 209).

**4.** Bone plate (101; 101'; 101'') according to one of the preceding claims, wherein the first receiving region (204; 364; 374; 384; 394) is designed for variable-angle fixation of the blocking element (310; 345; 355) in the first direction and the second receiving region (206; 366; 376; 386; 396) is designed for variable-angle fixation of the blocking element (310; 345; 355) in the second direction.

**5.** Surgical set, comprising at least one bone plate (101; 101'; 101''; 360; 370; 380; 390) according to one of the preceding claims and at least one bone screw (301; 340; 350) with a screw shank (320; 343; 353) and a locking element (310; 345; 355), in particular a screw head (310) projecting outwards beyond the screw shank (320) and a thread (321) of the screw shank (320; 343; 353), wherein the locking element (310; 345; 355) can be received selectively in the first receiving region (204; 364; 374; 384; 394) or in the second receiving region (206; 366; 376; 386; 396) of the opening (102, 102', 146; 361; 371; 381; 391) and in particular can be fixed at variable angles.

**6.** Surgical set according to claim 5, wherein the locking element (310; 345; 355) is provided with a circumferential outer surface which extends substantially in the direction of a longitudinal axis (K) of the bone screw (301; 340; 350) and has at least one clamping surface (330; 346; 356) which - viewed in an azimuthal plane perpendicular to the longitudinal axis (K) - widens outwards away from the longitudinal axis (K) in a wedge shape.

**7.** Surgical kit according to claim 6, wherein the circumferential outer surface of the blocking element (310; 345; 355) is at least approximately spherical, paraboloidal, ellipsoidal or hyperboloidal at least in the region of the clamping surface (330; 346; 356).

**Revendications**

**1.** Plaque d'ostéosynthèse (101 ; 101' ; 101'' ; 360 ; 370 ; 380 ; 390), contenant au moins une ouverture (102, 102', 146 ; 361 ; 371 ; 381 ; 391) destinée à recevoir respectivement au moins une vis à os (301 ; 340 ; 350),

l'ouverture (102, 102', 146 ; 361 ; 371 ; 381 ; 391) traversant la plaque d'ostéosynthèse (101 ; 101' ; 101'' ; 360 ; 370 ; 380 ; 390) le long d'un axe longitudinal (L) depuis une face supérieure (202 ; 362 ; 372 ; 382 ; 392) jusqu'à une face inférieure opposée (203 ; 363 ; 373 ; 383 ; 393), l'ouverture (102, 102', 146 ; 361 ; 371 ; 381 ; 391) sur la face supérieure (202 ; 362 ; 372 ; 382 ; 391) ; 392) débouchant dans une première zone de réception (204 ; 364 ; 374 ; 384 ; 394) qui est conçue pour recevoir et fixer un élément de blocage (310 ; 345 ; 355) d'une vis à os (301 ; 340 ; 350) dans une première direction, **caractérisé en ce que** l'ouverture (102, 102', 146 ; 361 ; 371 ; 381 ; 391) débouche sur la face inférieure (203 ; 363 ; 373 ; 383 ; 393) dans une deuxième zone de réception (206 ; 366 ; 376 ; 386 ; 396) qui est conçue pour recevoir et fixer l'élément de blocage (310 ; 345 ; 355) dans une deuxième direction, la deuxième direction étant sensiblement opposée à la première direction.

**2.** Plaque d'ostéosynthèse (101 ; 101' ; 101'') selon la revendication 1, la première zone de réception (204) étant délimitée par une première paroi intérieure (205), la deuxième zone de réception (206) étant délimitée par une deuxième paroi intérieure (207), au moins un évidement (208, 209) étant formé dans la première paroi intérieure (205) ainsi que dans la deuxième paroi intérieure (207), la distance de la paroi intérieure respective (205, 207) augmentant dans chacun de ces évidements (208, 209) en fonction de l'angle de rotation autour de l'axe longitudinal (L) .

**3.** Plaque d'ostéosynthèse (101 ; 101' ; 101'') selon la revendication 2, dans laquelle aussi bien la première paroi interne (205) que la deuxième paroi interne (207) sont au moins approximativement sphériques, paraboloïdes, ellipsoïdes ou hyperboloïdes dans la zone de chacun des évidements respectifs (208, 209).

**4.** Plaque d'ostéosynthèse (101 ; 101' ; 101'') selon l'une des revendications précédentes, dans laquelle la première zone de réception (204 ; 364 ; 374 ; 384 ; 394) est conçue pour la fixation à angle variable de l'élément de blocage (310 ; 345 ; 355) dans la première direction et la deuxième zone de réception (206 ; 366 ; 376 ; 386 ; 396) est conçue pour la fixation à angle variable de l'élément de blocage (310 ; 345 ; 355) dans la deuxième direction.

5. Ensemble chirurgical comprenant au moins une plaque d'ostéosynthèse (101 ; 101' ; 101'' ; 360 ; 370 ; 380 ; 390) selon l'une des revendications précédentes ainsi qu'au moins une vis à os (301 ; 340 ; 350) avec une tige de vis (320 ; 343 ; 353) et un élément de blocage (310 ; 345 ; 355), en particulier une vis de blocage (310 ; 345 ; 355) qui dépasse la tige de vis (320) et un filetage (321) de la tige de vis (320 ; 343 ; 353) vers l'extérieur, l'élément de blocage (310 ; 345 ; 355) pouvant être reçu au choix dans la première zone de réception (204 ; 364 ; 374 ; 384 ; 394) ou dans la deuxième zone de réception (206 ; 366 ; 376 ; 386 ; 396) de l'ouverture (102, 102', 146 ; 361 ; 371 ; 381 ; 391) et pouvant être fixé en particulier avec un angle variable.

6. Ensemble chirurgical selon la revendication 5, dans lequel l'élément de blocage (310 ; 345 ; 355) est pourvu d'une surface extérieure périphérique qui s'étend sensiblement dans la direction d'un axe longitudinal (K) de la vis à os (301 ; 340 ; 350) et qui présente au moins une surface de serrage (330 ; 346 ; 356) qui - vue dans un plan azimutal perpendiculaire à l'axe longitudinal (K) - s'élargit en forme de coin vers l'extérieur en s'éloignant de l'axe longitudinal (K) .

7. Ensemble chirurgicale selon la revendication 6, la surface extérieure périphérique de l'élément de blocage (310 ; 345 ; 355) étant au moins approximativement sphérique, paraboloïde, ellipsoïdale ou hyperboloïde au moins dans la zone de la surface de serrage (330 ; 346 ; 356).

**Figur 1**

**Figur 2**

**Figur 3a**

Figur 3b

Figur 4b

Figur 4a

**Figur 5b**

**Figur 5a**

Figur 6a

Figur 6b

**Figur 7**

Figur 8

Figur 9a

Figur 9b

Figur 9c

Figur 9d

Figur 9e

**Figur 9f**

101'

**Figur 9g**

160
161
164
131
101'

**Figur 9h**

101'

**Figur 9i**

101'

Figur 10

**Figur 11a**

**Figur 11b**

**Figur 12a**

**Figur 12b**

**Figur 13a**

**Figur 13b**

**Figur 13c**

**Figur 14a**

**Figur 14b**

**Figur 15a**

**Figur 15b**

**Figur 15c**

372   371   370

**Figur 16a**

372   371   370

**Figur 16b**

372  377   371   374   370

373   376   378

**Figur 16c**

**Figur 17a**

**Figur 17b**

**Figur 17c**

**Figur 18a**

**Figur 18b**

**Figur 18c**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004086990 A1
- US 2009281543 A1
- WO 0182809 A
- WO 0066012 A1
- WO 03068091 A1
- US 4726808 A
- RU 2033105 C1
- WO 2010080511 A1

- WO 2013096592 A1
- DE 10335281 A1
- EP 1182972 B1
- WO 2004086990 A
- CH 669105 A5
- CH 675531 A5